# EUROPÄISCHE PATENTSCHRIFT

(11) **EP 3 000 396 B1**
(45) Veröffentlichungstag und Bekanntmachung des Hinweises auf die Patenterteilung: **28.06.2017**
(21) Anmeldenummer: 15186553.2
(22) Anmeldetag: 23.09.2015
(51) Int. Cl.: A61B 5/11, A61B 5/00, G09B 19/00

(54) **TRAGBARES BEWEGUNGSANALYSESYSTEM**
PORTABLE MOTION ANALYSIS SYSTEM
SYSTEME PORTATIF D'ANALYSE DE DEPLACEMENT

(30) Priorität: 26.09.2014 DE 102014014017
(43) Veröffentlichungstag der Anmeldung: 30.03.2016
(73) Patentinhaber: Schindler, Hermann, 78532 Tuttlingen (DE)
(72) Erfinder: Schindler, Hermann, 78532 Tuttlingen (DE)
(74) Vertreter: LifeTech IP Spies & Behrndt Patentanwälte PartG mbB

(56) Entgegenhaltungen:
- US-A1- 2001 034 583
- US-A1- 2005 021 292
- US-A1- 2012 322 570
- US-B1- 8 573 982

## Beschreibung

Die vorliegende Erfindung bezieht sich auf ein tragbares Bewegungsanalysesystem, und insbesondere auf ein tragbares und in Sportgeräte integrierbares Sensorsystem zur Erfassung, Messung, Analyse, Überwachung und Verbesserung von Bewegungsabläufen. Weitere Aspekte der vorliegenden Erfindung beziehen sich auf eine technische Ausführung und eine Überwachung bzw. Verbesserung von realisierten Trainingsleistungen für Einzelsportarten, wie beispielsweise Laufen/Jogging, Ski alpin und Skilanglauf.

### Hintergrund

Viele Sportarten erfordern eine möglichst exakte Ausführung einer Sporttechnik, um einerseits die eigene Leistung zu verbessern, aber auch um Überlastungen zu vermeiden. Ungefähr 70% der Läufer oder Jogger bekommen mindestens einmal im Jahr Probleme im Fußbereich, wofür die Lauftechnik eine wichtige Ursache ist. Gerade im letzten Drittel des Trainings sinkt die Konzentration auf die Technik und Fehlbewegungen schleichen sich ein.

Sensorsysteme zum Erfassen, Aufzeichnen und Analysieren der Herzfrequenz, der zurückgelegten Wegstrecke (z.B. Bewegung des Sportlers insgesamt im Raum oder im Freien) und der Geschwindigkeit sind im Sport bereits bekannt. Auch das Erfassen der Schrittfrequenz und der Schrittlänge durch in Sportschuhe integrierbare Sensoren sind bekannt und beispielsweise in EP 2 650 807 A1 und US 2013/0274587 beschrieben. Das alleinige Erfassen und Auswerten der Schrittfrequenz und der Schrittlänge sagt noch nichts über die biomechanisch korrekte Führung und das Abrollen des Fußes aus. Auch die Erfassung der Kraft oder Leistung beim Abdruck des Fußes kann in diesen bekannten Systemen nicht genau ermittelt werden.

Ein weiteres konventionelles System ist in US 2013/0274635 offenbart, in welchem die Aktivitäten des Sportlers in Bezug auf das Sportgerät überwacht werden, wobei insbesondere die Wechselwirkung zwischen dem Sportler und einem Ball analysiert wird. Auch dieses System erlaubt es nicht, einen technisch bzw. biomechanisch korrekten Bewegungsablauf des Sportlers zu analysieren.

In US 8,762,101 und US 2013/0323438 werden Transfermöglichkeiten der erfassten Sensordaten für eine zurücklegte Wegstrecke, eine Geschwindigkeit, eine Herzfrequenz auf ein Online-Logfile oder in sozialen Medien beschrieben. Außerdem beschreibt US 8,749,380 einen Sensor in einem Schuh, der die zurückgelegte Wegstrecke misst und ein Signal ausgibt, wenn der Schuh ausgetauscht werden muss. In US 2005/0151350 ist ein Sensorsystem beschrieben, welches Skivibrationen misst und die Skihärte durch ein elektronisches Element entsprechend darauf anpasst. Für den spezifischen Bereich von Freestyle-Skifahren ist in US 8,239,146 und US 8,620,600 ein System beschrieben, welches neben der Geschwindigkeit, eine Messung der Zeit in der Luft (Flugzeit) und der Anzahl von Drehungen in der Luft erfasst. Dies kann durch ein vergleichsweise einfaches Sensorsystem erfasst werden, wie beispielsweise ein vorhandenes Smartphone.

US 8,573,982 B1 offenbart ein weiteres Verfahren zum Überwachen einer sportlichen Tätigkeit, wobei Sensordaten, die sich auf eine Bewegung des Oberkörpers des Nutzers beziehen, während einer Laufbewegung erfasst und analysiert werden, um unerwünschte Bewegungen des Oberkörpers zu vermeiden. US 2001/0034583 A1 offenbart eine Geschwindigkeitsmessvorrichtung für einen Sportler mit einem Sensor, der feststellt, wann der Sportler sich vom Boden abhebt und auf den Boden zurückkehrt. Die erfassten Daten werden ausgewertet und angezeigt. Außerdem kann ein Geschwindigkeitssensor die Geschwindigkeit erfassen und anzeigen. US 2005/0021292 A2 offenbart ein System zur Bestimmung von Leistungsdaten eines Sportlers, der einen Sensor trägt, um Daten wie die Geschwindigkeit, die Fallhöhe, die Zeit während eines Sprunges zu erfassen und auszuwerten.

Ein Nachteil der bekannten Systeme liegt daran, dass ein Bewegungsablauf des Sportlers im Allgemeinen nicht erfasst werden kann und diese Systeme sich nur begrenzt für Anwendungen einsetzen lassen, um die Effizienz des Sportlers zu erhöhen bzw. um Fehlhaltungen oder Fehlbewegungen des Sportlers zu vermeiden.

Der vorliegenden Erfindung liegt die Aufgabe zugrunde, ein Bewegungsanalysesystem zu schaffen, das es ermöglicht, eine Bewegungsabweichung eines Sportlers oder Nutzers von einem Bewegungsprofil zu erfassen und einen Bewegungsablauf zu analysieren.

Eine weitere Aufgabe der vorliegenden Erfindung besteht in einer komplexen Koordination von Bewegungsabläufen der Extremitäten (Füße, Beine, Arme etc.).

### Zusammenfassung

Die oben genannte Aufgabe wird durch ein Bewegungsanalysesystem nach Anspruch 1 und ein Verfahren nach Anspruch 14 gelöst. Die abhängigen Ansprüche beziehen sich auf vorteilhafte Weiterbildungen der Gegenstände der unabhängigen Ansprüche.

Der Begriff "tragbar" ist bei der vorliegenden Erfindung weit auszulegen und umfasst ebenfalls mobile Bewegungsanalysesysteme, die beispielsweise an oder in einem Ski integriert sind und nicht notwendigerweise durch den Nutzer während der Sportausübung getragen werden. Vielmehr soll tragbar als Gegensatz zu "stationär" verstanden sein und sich somit auf alle Freiluft Aktivitäten, die eine Ortsänderung umfassen, beziehen.

Optional ist in weiteren Ausführungsbeispielen die zweite Verarbeitungseinheit ausgebildet, eine Bestätigung für die Bewegungsabweichung von dem Bewegungsprofil bezüglich des ersten Sensormoduls und des zweiten Sensormoduls festzustellen und, in diesem Fall, eine Nachricht an das erste Sensormodul zu senden. Die erste Verarbeitungseinheit kann ausgebildet sein, die Nachricht des zweiten Sensormoduls auszuwerten und das Bewegungsprofil anzupassen.

Optional ist in weiteren Ausführungsbeispielen die erste Verarbeitungseinheit und die zweite Verarbeitungseinheit ausgebildet, um eine zeitliche Abfolge von Beschleunigungsdaten von dem ersten Beschleunigungssensor und/oder von dem zweiten Beschleunigungssensor zu erfassen. Darauf basierend kann eine Geschwindigkeit und/oder eine Richtungsänderung des Nutzers oder eines Sportgerätes oder einer Sportausrüstung festgestellt werden.

Optional umfassen in weiteren Ausführungsbeispielen das erste Sensormodul und/oder das zweite Sensormodul weiter zumindest eine der folgenden Komponenten: einen Datenspeicher, einen Energiespeicher, eine Nutzerschnittstelle, einen Drucksensor, einen Schwerkraftmesssensor, einen Temperatursensor, einen magnetischen Sensor, ein Gyroskop, einen Windsensor, einen Vibrationssensor, eine 3D-Kamera, einen Ultraschallsensor, eine Spektralanalyseeinheit, ein chemischer Analysesensor und eine Leitfähigkeitsmesseinrichtung.

Optional ist in weiteren Ausführungsbeispielen der Drucksensor des ersten Sensormoduls und/oder des zweiten Sensormoduls ausgebildet, um einen Druck zwischen einem Sportausrüstung oder einem Sportgerät des Nutzers und einem Untergrund zu messen, und wobei die erste Verarbeitungseinheit und/oder die zweite Verarbeitungseinheit ausgebildet sind, um den gemessene Druck mit entsprechenden Werten in dem Bewegungsprofil zu vergleichen.

Außerdem können optional insbesondere optisch, elastische Fasern oder andere textile Drucksensoren zur Druck-Messung (im folgenden Druck-Fasern) vorgesehen sein, die beispielsweise in die Ski und/oder deren Bestandteile eingewoben oder eingebracht werden können.

Optional ist in weiteren Ausführungsbeispielen die 3D-Kamera ausgebildet, um eine räumliche Abbildung einer Umgebung des ersten und/oder des zweiten Sensormoduls zu erzeugen. Die erste Verarbeitungseinheit und/oder die zweite Verarbeitungseinheit können ausgebildet sein, um eine räumliche Orientierung einer Sportausrüstung im Vergleich zu einem Untergrund festzustellen und Abweichungen von einem Normwert auszugeben.

Optional ist in weiteren Ausführungsbeispielen die erste Verarbeitungseinheit und/oder die zweite Verarbeitungseinheit ausgebildet, um basierend auf Sensordaten von zumindest einen Beschleunigungssensor, zumindest einem Gyroskop und/oder zumindest eine 3D-Kamara eine Traktion einer Sportausrüstung zu einem Untergrund festzustellen und Abweichungen von einem Normwert auszugeben.

Optional ist in weiteren Ausführungsbeispiele die erste Verarbeitungseinheit und/oder die zweite Verarbeitungseinheit ausgebildet, um aus Messwerten in Bezug auf physikalischen und/oder chemischen Eigenschaften, insbesondere von der Leitfähigkeitsmessreinrichtung, eine Gleitfähigkeit von einem Sportgeräten auf gefrorenem Wasser, insbesondere Schnee, zu ermitteln. Die erste Verarbeitungseinheit und/oder die zweite Verarbeitungseinheit können ausgebildet sein, die Gleitfähigkeitsdaten zu erfassen und davon abhängig das Bewegungsprofil anzupassen.

Allgemeiner können physikalische und/oder chemische Messwerte auf verschiedenste Art ermittelt werden. Zum Beispiel kann die elektronische Leitfähigkeitsmesseinrichtung eine Gleitfähigkeit von einem Sportgerät auf gefrorenem Wasser ermitteln. So lässt sich mit der Messung der elektrischen Leitfähigkeit beispielsweise ein (flüssiger) Wasseranteil bzw. darin gelöstes Salz ermitteln. Die chemischen Eigenschaften, können beispielsweise über ein Analysetool zu chemischen Eigenschaften bestimmt werden. Bestimmte chemische Stoffe können aber auch durch eine Spektralanalyse bestimmt werden.

Optional sind in weiteren Ausführungsbeispielen die erste Verarbeitungseinheit und/oder die zweite Verarbeitungseinheit ausgebildet, um aus Messwerten des Vibrationssensors einen Gleit- oder Rollwiderstand zu einem Untergrund zu erfassen.

Optional ist in weiteren Ausführungsbeispielen die zweite Datenübertragungseinheit ausgebildet, um ein Ergebnis der zweiten Verarbeitungseinheit dem Nutzer zu visualisieren und/oder in einem Hauptspeicher zu speichern und/oder zu einer Ausgabeeinheit zu senden.

Optional ist in weiteren Ausführungsbeispielen das erste Sensormodul und/oder das zweite Sensormodul an oder in einer Sportausrüstung angeordnet oder integriert.

Optional umfasst die Sportausrüstung zumindest eines aus dem Folgendem: ein Schuh, ein Langlaufski, ein Abfahrtsski, eine Skibindung, ein Teil einer Sporthose, ein Teil einer Sportjacke, ein Teil eines Skistocks.

Optional umfasst das erste Sensormodul und/oder das zweite Sensormodul ein Energie-Erzeugungsmodul, welches ausgebildet ist, um elektrische Energie aus Schwingungen während der Bewegung zu gewinnen.

Optional ist in weiteren Ausführungsbeispielen die erste Verarbeitungseinheit ausgebildet, um einen Bewegungsablauf eines Beines des Nutzers festzustellen, und/oder die zweite Verarbeitungseinheit ist ausgebildet, um einen Bewegungsablauf des anderen Beines des Nutzers festzustellen, und jeweils mit einer optimalen Form zu vergleichen und Abweichungen davon auszugeben.

Optional ist die zweite Verarbeitungseinheit weiter ausgebildet, um Sensordaten, die von dem ersten und/oder dem zweiten Sensormodul erfasst wurden, zur direkten oder indirekten Druckmessung zu nutzen, um so einen Gravitationsdruckpunkt bezüglich zumindest eines Beines zu ermitteln und in einem zeitlichen Verlauf zu erfassen und darauf basierend eine Druckpunktfunktion zu ermitteln, mit einem Profil zu vergleichen und Abweichungen davon auszugeben.

Optional ist in weiteren Ausführungsbeispielen die erste Verarbeitungseinheit und/oder die zweite Verarbeitungseinheit ausgebildet, um basierend auf den erfassten Daten ein für den Nutzer individuelles Soll-Bewegungsprofil zu erzeugen und für eine folgende Erfassung der Bewegungsabweichung des Nutzers zu verwenden. Die erfassten Daten können beispielsweise von zumindest einem der vorhandenen Sensoren kommen oder sind über die Nutzerschnittstelle von dem Nutzer eingegeben worden.

Optional ist in weiteren Ausführungsbeispielen die erste Verarbeitungseinheit und/oder die zweite Verarbeitungseinheit ausgebildet, um basierend auf den erfassten Daten ein Belastungsprofil verschiedener Körperregionen oder Muskelregionen des Nutzers zu erstellen.

Somit ist aufbauend auf dem erfassten Bewegungsablauf ein "individuell optimaler" Bewegungsablauf und eine entsprechende Steuerung von Präventionsprogrammen möglich. Als Beispiel für ein Belastungsprofil kann ein Muskel-/Rückenindex erstellt werden. Somit umfassen Ausführungsbeispiele ebenfalls die Möglichkeit, dass basierend auf den ermittelten Bewegungsdaten - aber auch weiterer manueller Eingabe- und Korrekturdaten - einen für das jeweilige Individuum passender optimalen Bewegungsablauf ermittelt werden kann. Außerdem kann aufbauend auf der gemessenen Bewegung die geleistete Muskelarbeit der verschiedenen Muskelgruppen diagnostiziert und in Form von Kennziffern (z.B. den Muskel- und Rückenindex) ausgeben werden.

Dies bietet den Vorteile, dass der "individuell optimale" Bewegungsablauf auch für eine Trainings-/Schulungsmethodik, basierend auf den optimalen theoretischen Bewegungsabläufen und den individuell erfassten Bewegungsabläufen, erstellt werden kann. Außerdem können Präventionsprogrammen aufbauend auf den erfassten Bewegungsablauf (Muskel-/Rückenindex) gesteuert werden.

Die vorliegende Erfindung bezieht sich auch auf ein Skipaar mit einem zuvor beschriebenen Bewegungsanalysesystem, wobei das erste Sensormodul (100) und das zweite Sensormodul (200) jeweils mit der Bewegung eines entsprechenden Beines des Nutzers koppelbar sind.

Die vorliegende Erfindung bezieht sich auch auf ein Laufschuhpaar mit einem zuvor beschriebenen Bewegungsanalysesystem, wobei das erste Sensormodul (100) und das zweite Sensormodul (200) jeweils mit der Bewegung eines entsprechenden Beines des Nutzers koppelbar sind.

Die vorliegende Erfindung bezieht sich auch auf ein Verfahren gemäß Anspruch 14.

Ausführungsbeispiele bieten weiter die folgenden Vorteile:
Es erfolgt eine unmittelbare Rückkopplung von Sensordaten über die exakte Lauftechnik an den Läufer beim Laufen oder als eine Analysemöglichkeit nach dem Training. Beispielsweise kann die exakte Lauftechnik hinsichtlich des Abdruckes, der Fußstellung in verschiedenen Phasen der Bewegung analysiert werden und die Rückkopplung an den Läufer kann über Töne oder auf eine Datenbrille erfolgen. Damit ergeben sich neue Möglichkeiten für ein Techniktraining oder ein Skischultraining. Darüber hinaus bieten Ausführungsbeispiele die Vorteile, dass nicht nur willentlich steuerbare Fehler in der Lauftechnik analysiert und ausgewertet werden können, sondern auch dass ein Zusammenspiel der körperlichen Gegebenheiten des Sportlers (wie beispielsweise seine Größe, sein Gewicht, seine Beinlänge, seine Wirbelsäulenkrümmung, etc.) mit einfließen und das Zusammenspiel mit dem Sportgerät analysierbar ist. Beispielsweise bieten Hersteller von Laufschuhen entsprechend unterschiedliche Pronationstypen/Dämpfungstypen, Langlaufskihersteller verschiedene (Vor)Spannungen für die Skier an. Jedoch ist es immer noch sehr schwierig, die passenden Sportartikel individuell zu finden. Obwohl Orthopäden und Sporthändler beispielsweise Laufbandtests mit Videoanalysen anbieten, ist es aufgrund der kurzen Zeit und einer Laborumgebung, wo beispielsweise ein Sportler gezielt auf die Lauftechnik achtet und eine nur kurze Zeitphase zur Verfügung gestellt wird, schwierig ein individuell richtiges Sportgerät oder Sportartikel zu finden.

Ausführungsbeispiele bieten den weiteren Vorteil, dass Sportartikelhersteller und Händler die Sportartikel genau nach dem Bedarf des einzelnen Sportlers ausrichten können, da dafür Bewegungsabläufe, die das Bewegungsanalysesystem gemäß der vorliegenden Erfindung erfasst bei der Nutzung der Sportgeräte analysiert werden können. Beispielsweise können Sportartikelhersteller dabei analysieren, wie oft ein Nutzer welchen Ski bei welchen Schneearten einsetzt, wie seine Technik und Kondition ist, wo seine Schwächen liegen und wann er auf welchen Ski umsteigen sollte. Dies kann letztendlich für die individualisierte Produktion von Skiern und Schuhen und anderen Sportgeräten genutzt werden.

Außerdem ist es möglich, dass durch Auswertungen der Bewegungsdaten nach dem Training der Sportler Hinweise bekommt, wie er sich besser, schneller, gelenkschonender etc. bewegen kann oder sollte. Durch geeignete digitale Sportbrillen können weiterhin Informationen in Echtzeit visualisiert werden. Hierbei ist es ebenfalls möglich, neue Kombinationen aus realem Sport und Videospielen zu ermöglichen. Die Möglichkeiten der Zusammenarbeit von Trainern und Sport/Skilehrern mit den Sportlern übersetzt sich ebenfalls deutlich.

### Kurzbeschreibung der Figuren

Die Ausführungsbeispiele der vorliegenden Erfindung werden besser verstanden von der folgenden detaillierten Beschreibung und den beiliegenden Zeichnungen der unterschiedlichen Ausführungsbeispielen, die jedoch nicht so verstanden werden sollten, dass sie die Offenbarung auf die spezifischen Ausführungsbeispielen einschränkt, sondern lediglich der Erklärung und dem Verständnis dienen.
- Fig. 1: zeigt ein Ausführungsbeispiel für ein Bewegungsanalysesystem gemäß einem Ausführungsbeispiel der vorliegenden Erfindung.
- Fig. 2: zeigt einen Grundaufbau eines Sensormoduls.
- Fig. 3: veranschaulicht eine Datenübertragung zwischen den verschiedenen Sensorsystemen/Sensormodule zur Auswertung der Bewegungsanalyse.
- Fig. 4: veranschaulicht ein Rückkopplungssystem zur Information des Nutzers bei festgestellten Abweichungen.
- Fig. 5: zeigt eine mögliche schematische Visualisierung für einen Sportler über festgestellte Abweichungen.
- Fig. 6: gibt eine schematische Darstellung des Bewegungsanalysesystems und einen Datenverlauf zur Analyse der Bewegung.
- Fig. 7: zeigt unterschiedliche Bewegungsabläufe währen des Skilanglaufens.
- Fig. 8: zeigt weitere Fehlhaltungen bei einer Skiführung und einem Laufschuh.
- Fig. 9: zeigt eine beispielhafte Anordnung das Bewegungsanalysesystem für Ski und Laufschuhe.
- Fig. 10: zeigt eine beispielhafte Anordnung eines Sensormoduls in einem Skischuh, Skibindung oder Laufschuh.
- Fig. 11: zeigt einen Bewegungsablauf während des Laufens eines Sportlers.
- Fig. 12: zeigt die Einsatzmöglichkeit des Beschleunigungssensors beim Skilanglauf.
- Fig. 13: zeigt eine beispielhafte Anordnung eines Sensormoduls in Befestigungsmaterialien.
- Fig. 14: zeigt eine beispielhafte Anordnung des Sensormoduls in einem Schuh.
- Fig. 15: veranschaulicht eine Nutzung des Bewegungsanalysesystems zur Optimierung einer Skigleitbewegung.
- Fig. 16: veranschaulicht eine Nutzung des Bewegungsanalysesystems zur Optimierung von klassischem Skilauf.
- Fig. 17: veranschaulicht weitere Details zur Optimierung einer klassischen Skilanglauftechnik.
- Fig. 18: veranschaulicht weitere Details zur Nutzung des Bewegungsanalysesystems zur Messung einer Abdrucktechnik beim Laufen.
- Fig. 19: veranschaulicht eine Nutzung des Bewegungsanalysesystems zur Optimierung von Ski alpin und Snowboardaktivitäten.
- Fig. 20-23: veranschaulichen das Gravitationsdruckpunktverfahren.

### Detaillierte Beschreibung

Die vorliegende Erfindung betrifft insbesondere ein tragbares oder mobiles oder in Sportgeräte integrierbares Sensorsystem zur Erfassung, Messung, Analyse, Überwachung und Verbesserung eines Bewegungsablaufes oder einer technischen Ausführung des Sportes. Insbesondere für Sportarten, bei denen der Abdruck und die exakte Stellung der Fuße oder Arme von großer Bedeutung für die Leistung und den Trainingsfortschritt des Sportlers sind, sind Ausführungsbeispiele der vorliegenden Erfindung einsetzbar, sind jedoch nicht darauf beschränkt. Insbesondere aber nicht ausschließlich betrifft die vorliegende Erfindung ein Sensorsystem für Bewegungsabläufe bei Sportarten wie Laufen/Jogging, Fahrradfahren, Ski alpin und Skilanglauf und kann weiterhin sich auf einen Einbau und das Anbringen für die folgenden Sportgeräte geeignet sein: Laufschuhe, Ski alpin und Skilanglauf. Ebenso ist es möglich, Ausführungsbeispiele zumindest teilweise in Stöcke, Schuhe, Helme, Brillen und Bekleidung zu integrieren. Weitere mögliche Sportgeräte umfassen Inline-Skates, Snowboards, Skateboards, Wake-, Surf- und Kiteboards..

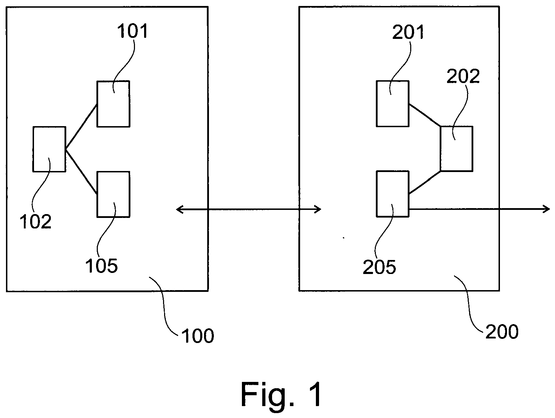

Fig. 1 zeigt ein tragbares Bewegungsanalysesystem zum Erfassen einer Bewegungsabweichung eines Nutzers von einem Bewegungsprofil, welches ein erstes Sensormodul 100 und ein zweites Sensormodul 200 umfasst.

Das erste Sensormodul 100 umfasst zumindest einen ersten Beschleunigungssensor 101, eine erste Datenübertragungseinheit 105 und eine erste Verarbeitungseinheit 102, wobei das erste Sensormodul 100 mit einem Körperteil des Nutzers koppelbar ist und ausgebildet ist, um erste Beschleunigungswerte des Körperteils zu erfassen. Das zweite Sensormodul 200 umfasst zumindest einen zweiten Beschleunigungssensor 201, eine zweite Datenübertragungseinheit 205 und eine zweite Verarbeitungseinheit 202, wobei das zweite Sensormodul 200 mit einem weiteren Körperteil des Nutzers koppelbar ist und ausgebildet ist, um zweite Beschleunigungswerte des weiteren Körperteils zu erfassen.

Die erste Verarbeitungseinheit 102 ist ausgebildet, um die erfassten Beschleunigungswerte von dem ersten Beschleunigungssensor 101 mit dem Bewegungsprofil zu vergleichen, und die erste Datenübertragungseinheit 105 ausgebildet ist, zumindest jene zu den ersten Beschleunigungswerten zugehörigen Daten an das zweite Sensormodul 200 zu übertragen, die von dem Bewegungsprofil abweichen. Die zweite Verarbeitungseinheit 202 ist ausgebildet, um die zweiten Beschleunigungswerte von dem zweiten Beschleunigungssensor 201 mit dem Bewegungsprofil zu vergleichen und mit den vom ersten Sensormodul 100 übertragenen Daten zu vergleichen und eine Bewegungsabweichung des Nutzers von dem Bewegungsprofil zu bestätigen und über die zweite Datenübertragungseinheit 205 auszugeben.

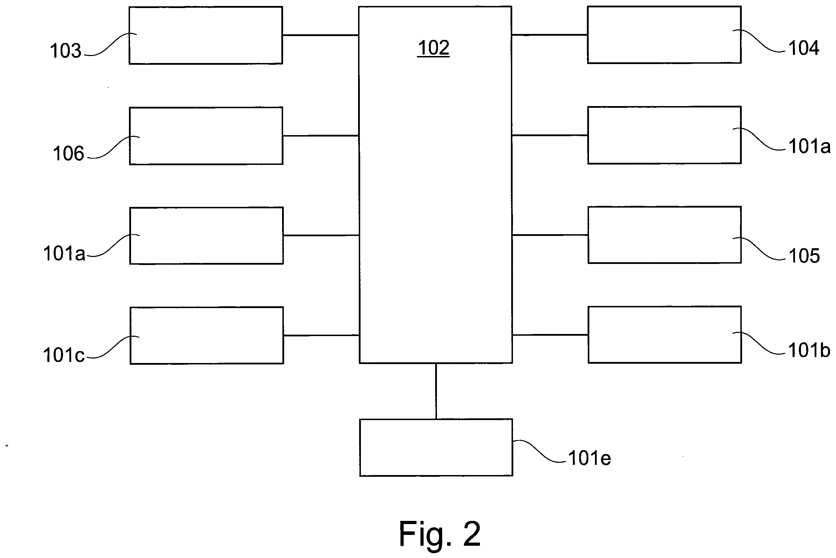

Fig. 2 zeigt einen Grundaufbau eines Sensormoduls 100 gemäß Ausführungsbeispielen der vorliegenden Erfindung. Das Sensormodul 100 weist einen oder mehrere Sensoren 101, einen Prozessor 102, eine Energiequelle 103, eine (optionale) Speichereinheit 104 und eine Datenübertragungseinheit 105 auf. Optional kann ebenfalls ein Ortungssystem oder ein Ortungssensor basierend beispielsweise auf GPS vorhanden sein (nicht gezeigt). Die einen oder mehreren Sensoren 101 umfassen einen Beschleunigungssensor 101a, einen Magnetfeldsensor 101b, eine 3D-Kamera 101c, einen Drucksensor 101d und weitere Sensoren 101e. Die weiteren Sensoren können beispielsweise ein Gyroskop, ein Temperatursensor, einen Schwerkraftmesssensor, einen Temperatursensor, einen Windsensor, einen Vibrationssensor, eine Leitfähigkeitsmesseinrichtung, ein chemischer Analysesensor, etc. umfassen. Der Beschleunigungssensor 101a kann beispielsweise ein Mehrachsen-Beschleunigungssensor umfassen (beispielsweise Drei-Achsen-Beschleunigungssensor). Weiter umfasst das Sensormodul, wie es in der Fig. 1 gezeigt ist, eine optionale Nutzerschnittstelle bzw. ein Bedienelement 106.

Die Sensoren 101, die Speichereinheit 104, die Energiequelle 103, die Datenübertragungseinheit 105 und die Nutzerschnittstelle 106 sind mit dem Prozessor 102 verbunden, in welchem Daten, wie sie durch die Sensoren erfasst und gespeichert in der Speichereinheit werden können, verarbeitet werden. Anschließend kann die Datenübertragungseinheit 105 die Daten übertragen. Die Datenübertragungseinheit 105 ist beispielsweise ein Übertragungsmodul welches Daten drahtlos überträgt. Optional können die Daten auch drahtgebunden übertragen werden.

Das Bewegungsanalysesystem umfasst als erstes und als zweites Sensormodul 100, 200 beispielsweise das Modul, wie sie in der Fig. 2 gezeigt ist. Daher können beide Sensormodule 100, 200 gleich aufgebaut sein, wobei die Funktionsweise sich durch eine entsprechende Programmierung des Prozessors wie zuvor beschrieben unterscheiden kann.

Das Bewegungsanalysesystem mit dem Sensormodul 100, wie es in der Fig. 2 gezeigt ist, kann für die folgend beschriebenen Bewegungsanalysen genutzt werden.

Wenn im Folgenden auf ein Sensorsystem Bezug genommen wird, kann es sich beispielsweise um einen Teil und ein vollständiges erstes Sensormodul 100 oder zweites Sensormodul 200 handeln. Es versteht sich, dass die Sensoren in dem Sensormodul verteilt auf einer Sportausrüstung angeordnet sein können, so dass ein Sensorsystem sich ebenfalls auf eine Untermenge der im ersten und/oder zweiten Sensormodul 100, 200 enthaltenen Sensoren und Komponenten handeln kann.

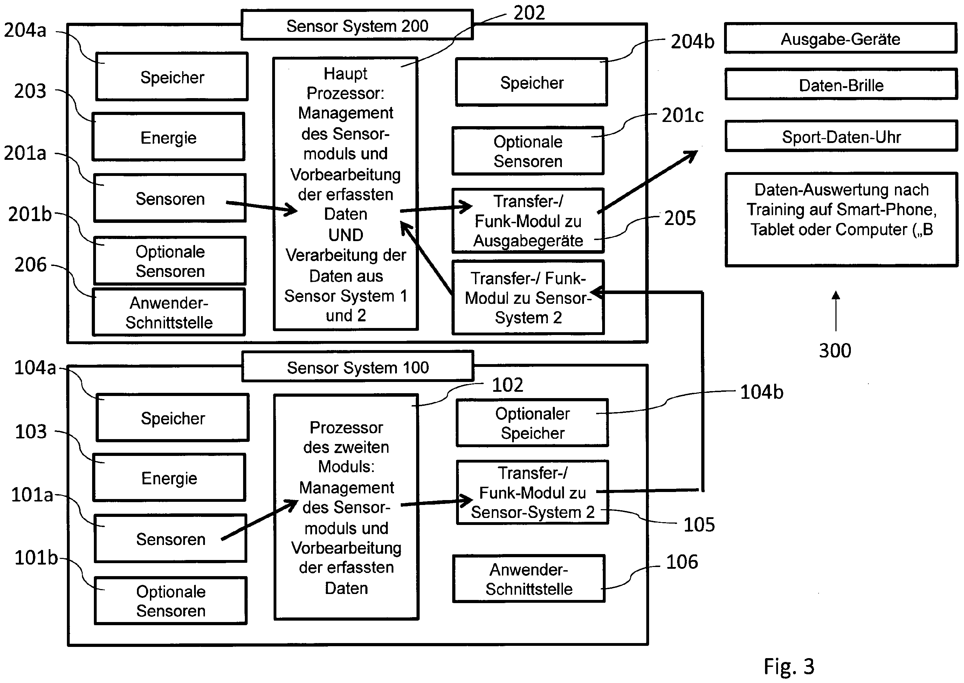

Fig. 3 zeigt ein Ausführungsbeispiel für das Bewegungsanalysesystem als ein integriertes System. Das Bewegungsanalysesystem weist ein zweites Sensorsystem 200 mit einem Hauptprozessor 202, einen ersten Speicher 204a und einen zweiten Speicher 204b, eine Energiequelle 203, einen ersten Sensor 201a, einen zweiten Sensor 201b, einen dritten Sensor 201c, wobei der zweite Sensor 201b und der dritte Sensor 201c optional sind und der/die Sensor(en) beispielsweise einen Mehrachsen-Beschleunigungssensor umfassen können. Ferner weist das zweite Sensorsystem 200 eine Datenübertragungseinheit 205 auf, die beispielsweise ein Transferfunkmodul zu einem Ausgabegerät umfassen kann. Ferner weist das zweite Sensorsystem 200 eine Anwenderschnittstelle 206 auf und ein Transferfunkmodul zu dem ersten Sensorsystem 100 auf.

Das erste Sensorsystem 100 weist ebenfalls einen Hauptprozessor 102 auf, einen Speicher 104a, einen optionalen Speicher 104b, eine Energiequelle 103, ein Transferfunkmodul 105 zu dem zweiten Sensorsystem 200 und eine Anwenderschnittstelle 106 auf. Außerdem ist ein erster Sensor 101a und ein zweiter Sensor 101b vorhanden, wobei der erste Sensor 101a wiederum ein Mehrachsenbeschleunigungssensor umfassen kann und der zweite Sensor 101b optional ist und weitere Sensoren umfasst sein können.

Das zweite Sensorsystem 200 ist beispielsweise ausgebildet eine Ausgabe zu einem Ausgabegerät zu übermitteln. Das Ausgabegerät 300 kann beispielsweise eine Datenbrille, eine Sportdatenuhr oder andere Datenauswertungseinrichtungen umfassen, wie beispielsweise ein Smartphone, ein Tablett oder ein Computer.

Das integrierte System, wie es in der Fig. 3 gezeigt ist, bietet folgende Funktionen. Viele Fehler oder Verbesserungsmöglichkeiten bei Bewegungen der Fortbewegung beruhen darauf, dass ein Fehler in einer Bewegung einen Fehler in den folgenden oder anderen Bewegungsschritten und vor allem eine Gegenbewegung provozieren kann. Die Gegenbewegung kann beispielsweise eine Bewegung des anderen Beines oder Armes sein. Die Korrektur des Fehlers muss bei der ursächlich falschen Bewegung und nicht bei den dadurch resultierenden falschen Bewegungsschritten ansetzen. Um ein Gesamtprofil der Bewegung erstellen zu können und die Interaktion der Teilbewegungen beurteilen zu können, reicht es nicht aus, die Differenz zweier Einzelmessungen von unabhängigen Sensormodulen zu analysieren. Vielmehr ist es erforderlich, diese Daten in einem direkten Zusammenhang miteinander zu bringen. Insbesondere ist es vorteilhaft, wenn die Daten in einem Raum- und Zeitbezug gesetzt werden. Eine Gesamtbeurteilung ist erst durch die Integration von einzelnen Werten in einem Gesamtzusammenhang sinnvoll möglich. Diese Funktionen werden durch das integrierte Sensorsystem, wie es in Fig. 3 gezeigt ist, ermöglicht.

Es sei darauf hingewiesen, dass alle Daten auf zwei unabhängige Einzelmodule gespeichert werden können und nachträglich zusammengeführt werden. Dies hat jedoch den Nachteil, dass sehr viele Einzeldaten in den Speichern gespeichert werden müssten und die Speicher- und Batteriekapazität schnell überlastet wären und dadurch das System langsamer und auch teurer werden würde. In diesem Fall wäre auch eine Echtzeitauswertung nicht möglich. Daher beruhen weitere Ausführungsbeispielen der vorliegenden Erfindung auf zwei Sensorsysteme, wobei Daten von Sensoren in einem der zwei Systemen erfasst und vorverarbeitet und aggregiert werden. Die Vorverarbeitung kann beispielsweise eine Datenkompression umfassen. Beispielsweise kann das Sensorsystem seine aggregierten und vorverarbeiteten Daten an das Hauptsystem 200 weitergeben. Im Hauptsystem 200 werden die vorverarbeiteten und aggregierten Daten vom System 100 und 200 zusammengeführt und ausgewertet. Danach kann die Information über die Bewegungsabläufe in Echtzeit auf Ausgabegeräte 300 wie beispielsweise eine Sportdatenuhr oder Datenbrillen gegeben werden oder auch gespeichert werden.

Diese Vorgehensweise für die Erfassung und Analyse von Bewegungsabläufen in einem synchronisierten und integrierten Sensorsystem bietet für die einzelnen Anwendungen die folgenden Vorteile.

Beispielsweise kann es bei der Anwendung von alpinen Skilaufen dazu kommen, dass nur ein Ski auf einer Eisplatte gelangt, was den Ablauf des zweiten Skis beeinträchtigen kann. Der zweite Ski wird jedoch kein Abdriften aufgrund der Eisplatte zeigen. Ein unabhängig operierendes zweites Skisensorsystem erfasst nur die eine falsche Bewegung und stellt dies als ein Fahrfehler des Sportlers dar. Eine solche Fehlinterpretation wird durch ein Bewegungsanalysesystem gemäß der vorliegenden Erfindung vermieden.

In ähnlicher Weise kann es beim Laufen dazu kommen, dass der Abdruck eines Beines nicht optimal erfolgt und die Flug- und Landephase des anderen Fußes beeinträchtigt wird. Beispielsweise kann der Fuß leicht nach außen gestellt sein beim Abdruck und so der Abdruck mit geringen oder falschen Abdruckimpuls erfolgen. Es ist ebenfalls möglich, dass die Neigung des Fußes beim Aufsetzen nicht der für den Sportler optimale Neigung entspricht, wobei die Muskeln versuchen werden dann diesen Fehler beim Abrollen des Fußes auszugleichen, wodurch es zu weiteren Fehlern bzw. Überreaktion kommen kann.

Beim Skilauf kann es dazu kommen, dass der Ski nach dem Abstoßen beim Skilanglauf klassisch nicht richtig nach hinten auspendelt, was dazu führen kann, dass erfolgskritische Abdruckzeitpunkte beim anderen Bein nicht richtig gesetzt werden. Auch hier bietet das vorliegende System den Vorteil, dass eindeutig diese Fehler identifizieren werden und den Sportlern angezeigt werden können. Optional werden weitere Sensorsysteme in Armen, Oberkörper, Knie etc., hinzugefügt, welches die Analysemöglichkeiten hinsichtlich der Gesamtbewegung des gesamten Körpers noch weiter verbessert und eine exakte Fehler-Ursache-Analyse ermöglicht wird.

Eine wichtige Komponente des Sensorsystems ist die Rückkopplung der erfassten Sensordaten an den Sportler in einer geeigneten Form. Insbesondere sind Ausführungsbeispiele dafür geeignet, eine Echtzeitrückkopplung zu ermöglichen. Bei weiteren Ausführungsbeispielen ist es jedoch ebenfalls möglich, eine Auswertung nach dem Training durchzuführen, um beispielsweise komplexe Daten des Sensorsystems automatisch in eine für den Sportler neuronal einfach auf- und verarbeitbare Information zu bringen.

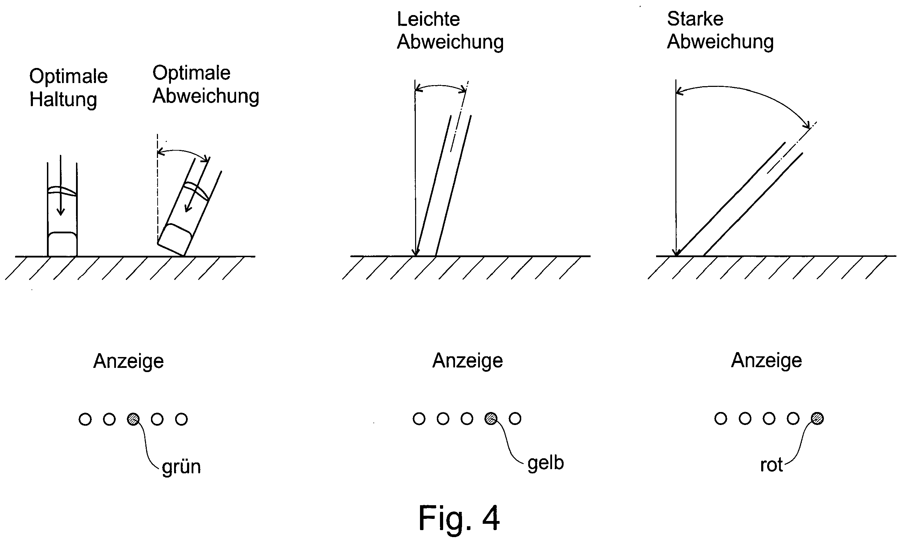

Fig. 4 zeigt ein Ausführungsbeispiel für eine mögliche Implementierung, bei der eine Abweichung der Ist-Bewegung von der optimalen Bewegung gemessen wird. Die Abweichung kann in numerischen Werten verschiedener Form an den Sportler zurückgekoppelt werden. Zum Beispiel zeigt die Fig. 4 einen Skischuh in einer optimalen Haltung und eine optimale Abweichung, die beispielsweise in einem Winkel im Grad ausgedrückt werden kann. Wenn diese optimale Haltung vorhanden ist, kann beispielsweise ein Rückkoppelsignal in Form einer grünen Farbe oder eines grünen Punktes erfolgen. Bei leichten Abweichungen von der optimalen Haltung, beispielsweise einer senkrechten Haltung des Skischuhs, erfolgt eine Ausgabe als gelbes Licht, wobei das gelbe Licht beispielsweise rechts vom grünen angeordnet sein kann und eine leichte Abweichung nach rechts signalisiert bzw. links vom grünen Punkt angeordnet sein kann, um eine leichte Abweichung nach links zu signalisieren. Bei starken Abweichungen kann stattdessen ein rotes Warnlicht erscheinen, wobei wiederum das rote Warnlicht auf der rechten Seite vom grünen Punkt eine starke Abweichung nach rechts anzeigen kann und eine rote Warnlicht auf der linken Seite eine starke Abweichung nach links von der optimalen Haltung anzeigen kann.

Sportliche Betätigungen zeichnen sich häufig dadurch aus, dass eine hohe Dynamik herrscht und viele Umweltreize aufgenommen und verarbeitet werden müssen. Das Sensorsystem gemäß Ausführungsbeispielen setzt hier an, indem Soll-Ist-Abweichungen erfasst werden, die ansonsten nicht in dieser Form erfasst werden können oder aufgrund der Informationsüberflutung untergehen. Dies bedeutet, dass die Information gerade bei einer Echtzeitrückkopplung leicht erfassbar und verarbeitbar sein sollte.

Ausführungsbeispiele der vorliegenden Erfindung ermöglichen dies beispielsweise durch die Visualisierung mittels verschiedenen Formen von "Ampeln". Beispielsweise können über LEDs verschiedene Farben ausgeben werden, wobei die Farben einen Grad von Misshaltung bzw. Abweichung von einer optimalen Haltung signalisieren. Beispielsweise kann die in der Fig. 4 gezeigte Fünfer-Ampel genutzt werden, um über eine rot-gelb-grün-gelb-rot-Lichtsequenz ein Abweichungsgrad entweder nach rechts oder links von der optimalen Haltung zu signalisieren. Ebenso möglich ist es Abweichungen nach oben/unten und/oder hinten/vorne schnell und einfach zu visualisieren.

Bei weiteren Ausführungsbeispielen ist es ebenfalls möglich eine Rückkopplung für den Sportler mittels komprimierter Kennziffern zu geben. Beispielsweise kann eine Gesamtnote für den Bewegungsablauf, eine Tachometeranzeige und eine Füllstandsanzeige für die Rückkopplung hinsichtlich prozentualer Werte oder absolute Abweichungswerte genutzt werden.

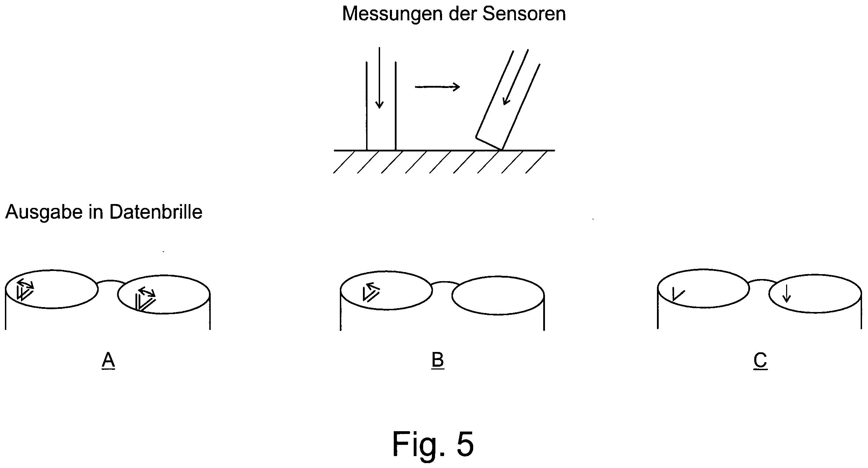

Fig. 5 zeigt eine Visualisierung in einer Datenbrille (beispielsweise mittels eines Overhead-Displays). Dazu ist oben in der Fig. 5 gezeigt, dass von einer optimalen senkrechten Haltung abgewichen wird in eine geneigte Haltung. Die Ausgabe in der Datenbrille zeigt an, in wieweit die geneigte Haltung dem Optimalwert entspricht. Dazu kann zunächst eine schematische Visualisierung genutzt werden, wie sie unter "A" gezeigt ist. Außerdem ist es möglich, Korrekturhinweise bzw. Verbesserungsvorschläge in Schrift und Bild zu zeigen. Diese Möglichkeit ist unter "B" gezeigt, wobei die geneigte Haltung mit einem Pfeil angedeutet wird bzw. eine Korrektur in die Pfeilrichtung vorgeschlagen wird. Schließlich ist es möglich, Korrekturhinweise mittels Symbole zu geben, wie sie beispielsweise unter "C" gezeigt sind. Einfache Pfeile können andeuten, dass entweder eine Bewegung nach rechts, links, oben, unten erfolgen sollte.

Bei komplexen Bewegungsabläufen ist es wegen der Informationsflut häufig nicht sinnvoll alle Abweichungen oder Teilbewegung zurück zu koppeln. Zum einen kann der Sportler seine Bewegung auswählen und/oder das System wählt die wichtigsten Bewegungen aus. Beispielsweise kann die ausgewählte Bewegung des Sportlers während des Trainings auch geändert werden. Kriterien für die Auswahl der Bewegung durch das System können beispielsweise sein: ein größter Fehler, Bedeutung für den Vortrieb/Erfolg.

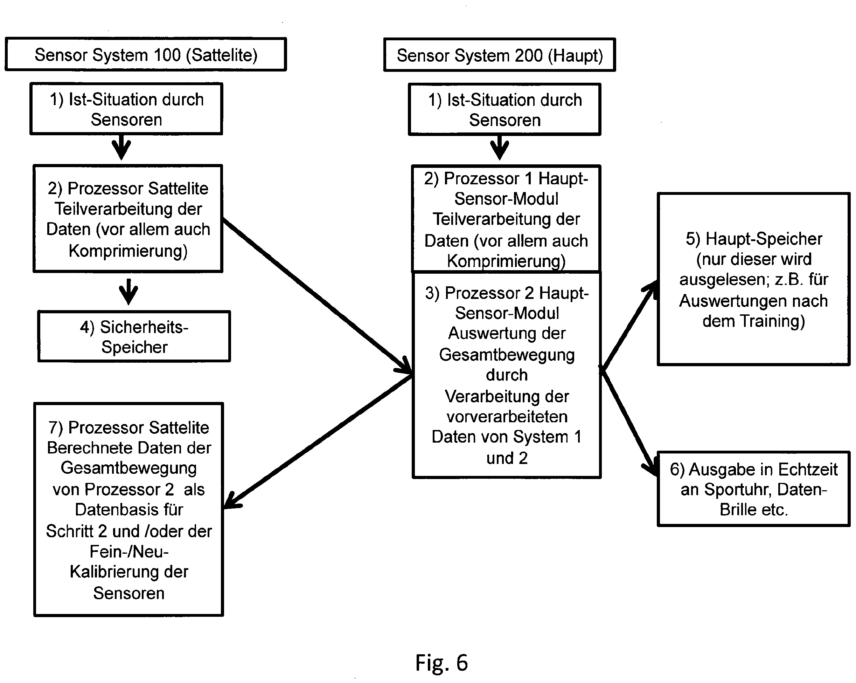

Fig. 6 gibt eine schematische Darstellung für die Auswertung der Sensordaten. Wiederum weist das Bewegungsanalysesystem ein erstes Sensorsystem 100 und ein zweites Sensorsystem 200 auf. In dem in der Fig. 6 gezeigten Ausführungsbeispiel ist das zweite Sensorsystem 200 das Hauptsystem, während das erste Sensorsystem das Satellitensystem ist.

Das Satellitensystem 100 erfasst zunächst die Ist-Situation durch Sensoren und gibt diese Sensordaten an den Prozessor des Satellitensystems weiter. Dort wird eine Teilverarbeitung durchgeführt, die beispielsweise eine Komprimierung der Sensordaten umfassen kann. Die vorverarbeiteten Sensordaten werden dann an das Hauptsystem 200 übermittelt.

Das Hauptsystem 200 ist ebenfalls ausgebildet, zunächst eine Ist-Situation über die vorhandenen Sensoren durchzuführen. Das Hauptsystem 200 umfasst wiederum einen Prozessor, der die Ist-Daten empfängt und eine Teilverarbeitung durchführt, die wiederum eine Datenkomprimierung umfassen kann. Außerdem ist der Prozessor des Hauptsystems 200 ausgebildet, die Gesamtauswertung durchzuführen, indem die vorverarbeiteten Daten aus dem Satellitensystem 100 und dem Hauptsystem 200 zusammen verarbeitet werden. Das Ergebnis der Gesamtauswertung kann anschließend an einen Hauptspeicher ausgegeben werden, wobei nur diese ausgelesen wird.

Die Auswertung kann optional auch nach dem Training erfolgen. Optional ist ebenfalls möglich, eine Echtzeitausgabe bereitzustellen, die beispielsweise über Sportuhren oder Datenbrillen erfolgen kann.

Optional ist es bei weiteren Ausführungsbeispielen ebenfalls möglich, dass das Hauptsystem nach der Gesamtauswertung das Ergebnis oder Teile davon zurückübermittelt an das Satellitensystem 100. Das Satellitensystem 100 kann dann durch den Prozessor die berechneten Daten der Gesamtbewegung von dem Prozessor des Hauptsystems 200 als Datenbasis für eine erneute Durchführung des Verarbeitungsschrittes 2) nutzen und/oder eine Fein- oder Neukalibrierung der Sensoren durchführen. Optional wird auch das Satellitensystem die vorverarbeiteten Daten nicht nur an das Hauptsystem übertragen, sondern diese in einem Speicher sichern.

Die Daten des Sicherheitsspeichers werden somit in der Regel nicht ausgelesen und dienen nur zur Absicherung und als Datenbasis für weitere Vorverarbeitungen innerhalb des Satellitensystems 100. Stattdessen brauchen nur die Ergebnisse des Hauptsystems in dem Hauptspeicher gespeichert bzw. an den Sportler übermittelt werden. Die Prozessoren vom Satellitensystem 100 und vom Hauptsystem 200 können eine physikalische Einheit sein oder zwei verschiedene Prozessoren sein.

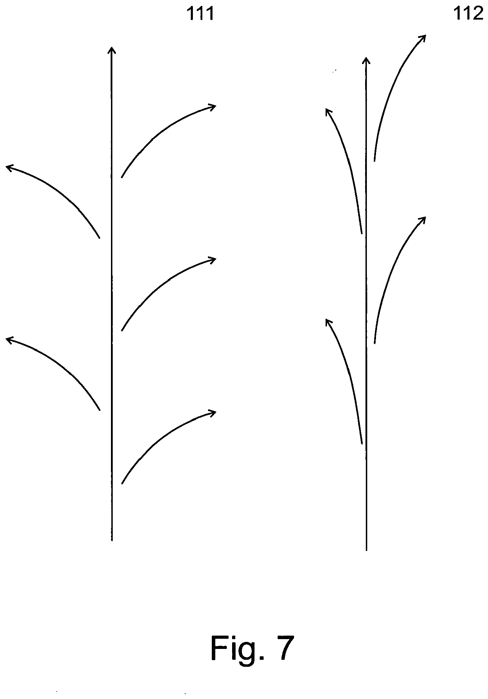

Fig. 7 zeigt eine beispielhafte Nutzung zur Analyse eines Langlaufskis. Das Bewegungsanalysesystem ist beispielsweise in der Lage, eine exakte Bewegung während des Skilanglaufens zu erfassen. Beispielsweise kann während eines Skating-Skilanglaufs eine Bewegung 111 mit weit geführten Ski erfolgen, wie es auf der linken Seite zu sehen ist. Bei dieser Bewegung 111 wird viel Energie verloren gehen. Andererseits ist es ebenfalls möglich, eine enge Skiführung 112 zu nutzen, wo eine Optimierung hinsichtlich der Leistung und des Vortriebes bzw. der Geschwindigkeit durchgeführt wurde.

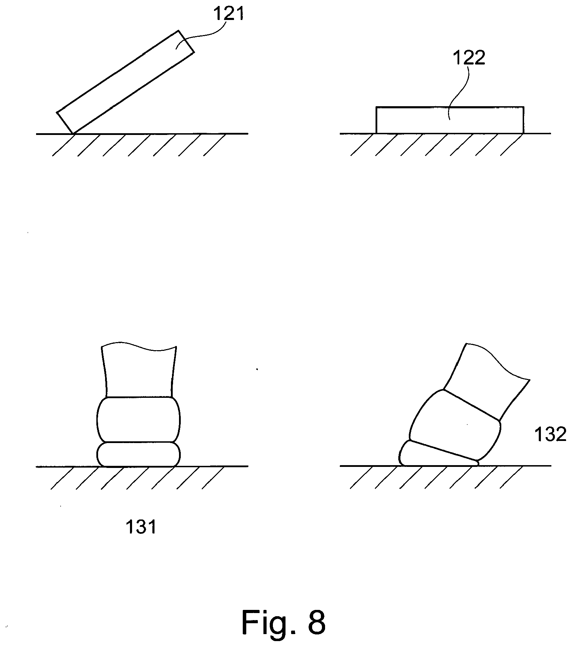

Fig. 8 zeigt als eine weitere Einsatzmöglichkeit des Bewegungsanalysesystems eine Analyse der Neigung von Sportgeräten. Beispielsweise kann in der oben gezeigten Darstellung analysiert werden, ob der Ski verkantet ist 121 oder ob der Ski flach auf einen Untergrund aufsetzt 122. In gleicher Weise ist es möglich (siehe unteren Teil der Fig. 7) festzustellen, ob ein Laufschuh flach aufsetzt 131 oder ein Laufschuh schräg aufgesetzt wird 132.

Um die in den Fig. 2 und 7 gezeigten Anwendungen zu ermöglichen, kann ein Abgleich von Daten, Bewegungen, Positionen zwischen beiden Schuhen/Ski, d.h. insbesondere mit dem korrespondierenden zweiten Schuh oder Ski, erfolgen. Dabei können beispielsweise folgende Sensoren zum Einsatz kommen. Einerseits werden Sensoren genutzt zur exakten Erfassung der Bewegung des Skis/Schuhs oder von Skistöcken und der Position(en). Hierfür kann beispielsweise der Mehrachsenbewegungssensor und/oder der Beschleunigungssensor, aber auch der MEMS Magnetfeldsensor oder das Gyroskope genutzt werden. Die Sensoren können beispielsweise dezentral/verteilt angeordnet sein, d.h. sie können auch außerhalb einer Haupteinheit angeordnet sein (d.h. sie brauchen nicht in einem gemeinsamen Gehäuse untergebracht zu sein) und sind mit einem Funkmodul oder Kabel miteinander verbunden.

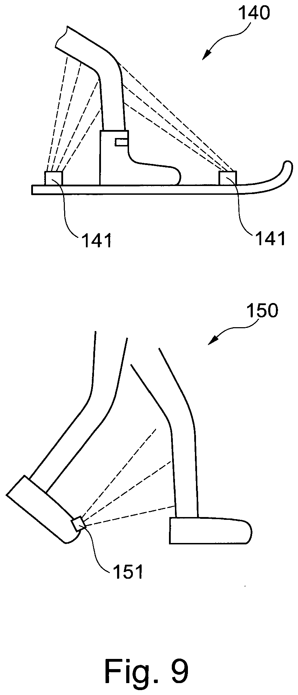

Fig. 9 zeigt eine weitere Einsatzmöglichkeit des Bewegungsanalysesystems für das Skilaufen 140 und das Laufen 150.

Beim Skilaufen 140 sind zwei Sensoren 141 an einem Ski verteilt angeordnet. Hierbei werden die Sensoren 141 genutzt, um eine Umgebung zu erfassen. Beispielsweise kann der Skiläufer sich an einem Skihang befinden. Dies kann durch eine Erfassung von Bewegungen und Haltungen des Knies durch eine 3D-Kamera 101c bei Ski alpin festgestellt werden. Die Sensoren 141 gehören zu einem Sensormodul 100, 200 mit zwei 3D-Kameras 101c. Analog kann beim Skilanglauf und Inline-Skating ein Sensor 141 zum Einsatz kommen. Der Sensor 141 kann z.B. einen Ultraschallsensor umfassen, der ebenfalls ausgebildet ist, um eine Entfernung zwischen einem Fuß und dem anderen Bein oder Knie zu bestimmen.

Beim Laufen 150 kann eine ähnliche Funktionalität dadurch erreicht werden, dass die Position und die Bewegung des zweiten Fußes oder Beines durch ein am Schuh oder im Schuh angebrachten Sensor 151 zur Erfassung des anderen Fußes oder Armes vorgesehen ist. Der Sensor 151 kann beispielsweise wiederum eine 3D-Kamera umfassen. Diese Analyse der Umgebung kann beispielsweise beim Laufen dazu genutzt werden, um die Bewegung des zweiten Fußes in der Luft und beim Abdruck oder eine Schlenkerbewegung in der Luft festzustellen oder um festzustellen, ob der Läufer auf ebenem oder unebenem Gelände, berghoch oder bergab läuft.

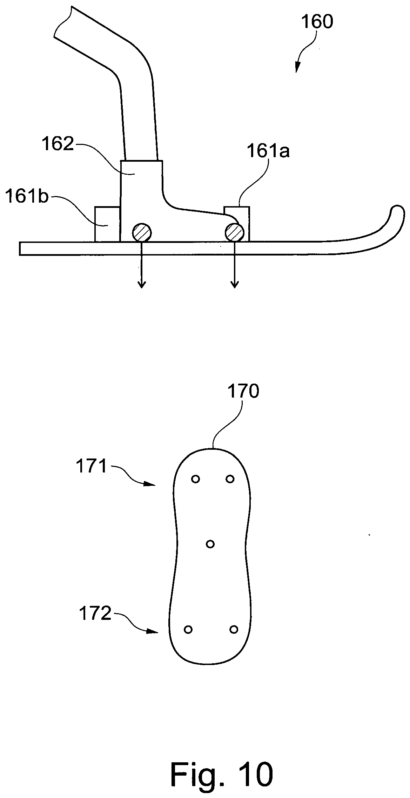

Fig. 10 zeigt ein weiteres Ausführungsbeispiel, bei welchem das erste Sensormodul 100 und/oder das zweite Sensormodul 200 einen Drucksensor 101d aufweist, um einen Druck zu ermitteln, den der Sportler auf das Sportgerät ausübt. Dieser Druck kann beispielsweise ein Abdruck sein, eine Gewichtsverlagerung, eine Druckverteilung bei einer Abrollbewegung, ein Aufprall beim Laufen oder Landen, ein Abstoßen oder Abdrücken bei Sportarten mit Stöcken. Der Drucksensor 101d kann beispielsweise eine Druckmessung vornehmen, die auf dem Ski beim Ski alpin wirkt, wobei der Drucksensor 101 d beispielsweise in der Bindung vorne 161 a oder hinten 161b angeordnet sein kann. Ebenso kann der Drucksensor 101d auch im Skischuh 162 angebracht werden.

Außerdem zeigt die Fig. 10 eine Einlegesohle 170, die beispielsweise in den Skischuh 162 einlegbar ist und einen Zehenbereich 171 und einen Fersenbereich 172 aufweist. Drucksensoren 101d können ebenfalls in der Einlegesohle 170 angeordnet sein, um so eine Druckverteilung in der Einlegesohle zu ermitteln. Zur Ermittlung der Abrollbewegung oder des Abdrucks beim Schuh oder der Sohle sind in der Regel mehrere Sensoren erforderlich. Aus biomechanischen und wirtschaftlichen Gesichtspunkten sind beispielsweise fünf, sieben oder neun Sensoren besonders geeignet.

Die genutzten Drucksensoren können Sensoren sein, die die Kraftwirkung exakt oder in einer Bandbreite messen. Optional können die Drucksensoren nur zwischen einer Belastung und Entlastung unterscheiden. Zur Verfügung stehende Drucksensoren reichen von Dehnungsmessstreifen, Piezosensoren bis hin zu Bekleidungsstoffen oder Bekleidungsgarnen, die eine Druckverteilung messen können.

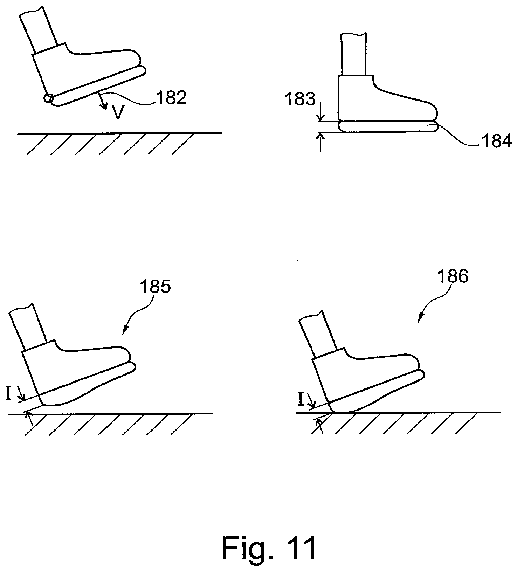

Fig. 11 veranschaulicht die Nutzung und Arbeitsweise des zumindest einen Beschleunigungssensors 101a, der beispielsweise als Drei-Achsen-Beschleunigungssensor oder als ein MEMS-Sensor ausgebildet ist. Insbesondere in Kombination mit weiteren Informationen über die Härte eines Skis, das Dämpfungsmaterial des Schuhs, kann der Beschleunigungssensor 101a genutzt werden zur indirekten Berechnung der Kraftwirkung.

Gezeigt ist beispielsweise ein Laufschuh 180, wobei an der Ferse 181 des Laufschuhs oberhalb der Dämpfungssohle ein Mehr-Achs-Beschleunigungssensor 101a und/oder ein Gyroskop und/oder ein Magnetfeldsensor 101b angebracht sind. Durch diese Sensoren ist es möglich, die Geschwindigkeit des Fußes in der Luft 182 zu erfassen, wobei die Höhe der Sohle 183 und der Härtegrad 184 der Sohle bekannt sind bzw. durch einen Nutzer eingegeben werden können. Beim Bodenkontakt des Fußes 185 wird der Fuß durch das Dämpfelement in der Schuhsohle weiter abgebremst und kommt schließlich zum Stillstand 186. Der Verlauf des Abbremsvorgangs in Kombination mit dem Härtegrad des Sportgeräts gibt indirekt Informationen über die Kraftwirkung.

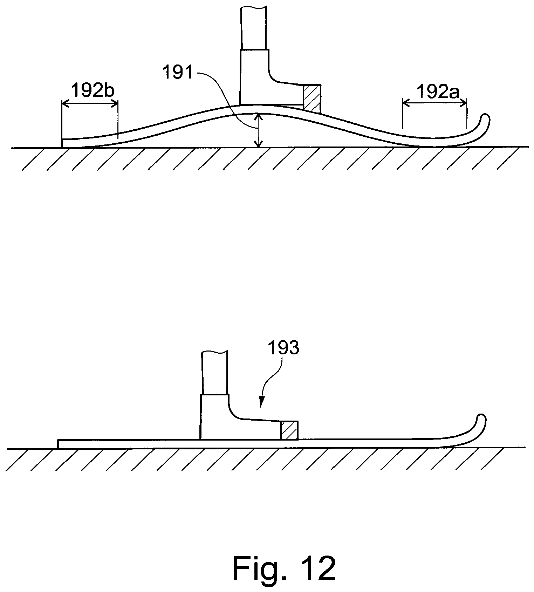

Fig. 12 zeigt die Einsatzmöglichkeit des Beschleunigungssensors 101a beim Skilanglauf klassisch. Der Ski hat im Allgemeinen eine sogenannte Vorspannung. Somit ist der Ski derart geformt, dass der mittlere Skibereich in dem Fall, dass kein Schneedruck besteht, eine mittlere Höhe 191 zum Schnee aufweist und lediglich der vordere und hintere Bereich 192 in Kontakt mit dem Schnee stehen, wenn der Läufer darauf gleitet oder mit beiden Beinen gleichmäßig darauf steht. Die Vorspannung soll verhindern, dass das sogenannte Steig- oder Haftwachs den Gleitvorgang bremst. Erst wenn der Läufer sich mit einem Bein abstoßen möchte, drückt der Ski nach unten, um das Haft- oder Steigwachs in Kontakt mit dem Schnee zu bringen, wobei das Wachs sich mit dem Schnee wie bei einem Klettverschluss verbindet und daran haften bleibt 193.

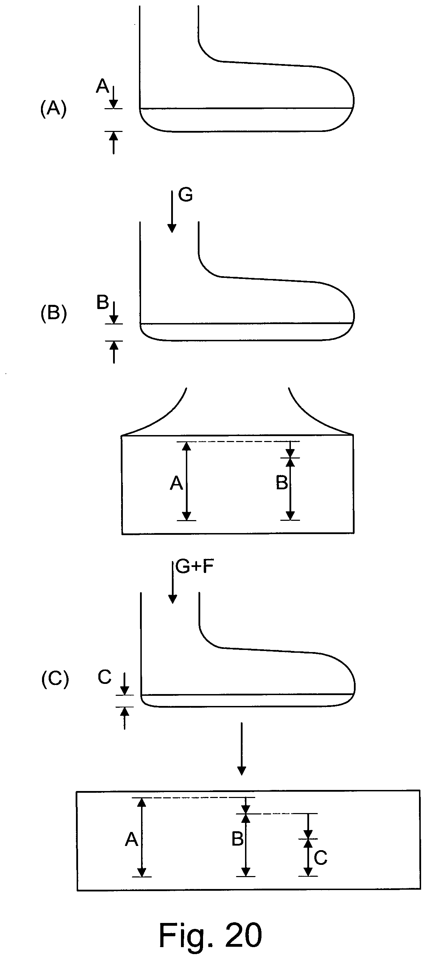

Dieser Kraftimpuls wirkt senkrecht nach unten und gibt eine wichtige Information für eine effiziente, korrekte Laufbewegung. Allerdings ist der Kraftimpuls an sich nicht so entscheidend, sondern vielmehr die durch die Sensoren gemessene Geschwindigkeit und wie stark sich der Ski nach unten verformt. Das Zusammendrücken des Dämpfelementes oder der Skispannung kann mittels einer Bewegungs- und Geschwindigkeitsmessung sehr genau erfasst werden und wird im Folgenden als Gravitationspunkt-Verfahren bezeichnet. Dieses Verfahren wird im Zusammenhang mit den Fig. 20 bis 23 im Detail erläutert.

Ein wichtiger Bestandteil für das Verfahren zur Erfassung des Bewegungsablaufs gemäß der vorliegenden Erfindung ist ein möglichst exakt dimensioniertes und am richtigen Ort des Sportgerätes platziertes Sensorsystem (d.h. die Sensoren der Sensormodule 100, 200). Darüber hinaus ist aber auch die Entwicklung und Verfeinerung eines geeigneten Algorithmus wichtig. Daraus lassen sich wertvolle Informationen bezüglich einer Krafteinwirkung ableiten, ohne die Kraft tatsächlich messen zu müssen. Bei vielen Anwendungen oder Sportarten ist die Kraft allein weniger oder nicht aussagekräftig.

Weitere Ausführungsbeispiele beziehen sich auf weitere Sportarten und Bewegungsarten, die hier nicht weiter beschrieben werden.

Weitere Sensoren der Sensormodule 100, 200 können genutzt werden, um einen Widerstand (oder andere Ergebnis-mindernde Einflüsse) beim durch den Sport erzielten Vortrieb zu erfassen. Gegenwind beim Laufen, rauer Straßenbelag beim Inline-Skaten, "stumpfe" Schnee beim Ski hindern beispielsweise den Vortrieb und die erzielte Leistung. Deshalb können Trainingsleistungen nicht verglichen werden. Eine Erfassung der Ergebnis-mindernden Faktoren kann die Qualität der Bewegungs- und Trainingsanalyse wesentlich verbessern. Ein weiterer Ergebnis-mindernder Faktor kann auch ein Wegrutschen durch eine Wahl der falschen Sohle/Dämpfung beim Laufen, des Falschen Steig-/Haftwachses bei Skilanglauf oder das seitliche Wegdriften beispielsweise durch falsche Räder beim Inline-Skaten sein. Sensoren können beispielsweise weitere Hinweise bezüglich des Rollwiderstandes oder des Schneewiderstandes liefern.

Ein wichtiger Nutzen der Ausführungsbeispiele der vorliegenden Erfindung liegt darin, dass die Sportgeräte mit den geringsten bremsenden Widerstand ausgewählt werden können. Beispielsweise kann der schnellste Ski für eine jeweilige Schneebedingung, ein schnellstes Rollmaterial (Rädern, Felgen, etc.) für den jeweiligen Straßenzustand, die beste Sohle oder Dämpfung von Laufschuhen ausgewählt werden.

Die hierfür genutzten Sensorsysteme können beispielsweise wie folgt ausgewählt werden (d.h. die Sensormodule 100, 200 umfassen die entsprechende Kombination von Sensoren):
Zur Messung des Windwiderstandes für alle vortriebsorientierten Freiluftsportarten wie beispielsweise Laufen, Skifahren, Skilanglauf, Radfahren können entsprechende Sensoren und miniaturisierte Messgeräte vorhanden sein. Die vom Sportler erzielte Leistung wird entsprechend um den Wind-Faktor korrigiert. Diese korrigierte Leistung kann in Relation zum erfassten Bewegungsablauf und weiterer Daten wie Herzrate weiteren Aufschluss über Verbesserungspotentiale beim Bewegungsablauf geben.

Die Gleitfähigkeit von Sportgeräten zum Gleiten auf Wasser in gefrorenem Zustand (Schnee oder Eis) kann beispielsweise durch die Erfassung von physikalisch-chemischen Parametern (beispielsweise durch eine elektrische Leitfähigkeitsmessung oder eine Spektralanalyse) ermittelt werden. Dies ist insbesondere vorteilhaft für Sportarten wie Ski alpin, Freestyle-Skilaufen, Langlauf aber auch für Snowboard und Schlittschuhlaufen. Durch den Gleitvorgang der Sportgeräte auf Schnee oder Eis entsteht ein dünner Wasserfilm zwischen der Gleitfläche der Sportgeräte (z.B. Skibelag oder Kufe), und dem Schnee oder Eis. Durch Erfassen der physikalisch-chemischen Parameter kann die Gleitfähigkeit der Sportgeräte bestimmt werden. Ein mögliches Verfahren ist beispielsweise die direkte Sensormessung in der Lauffläche von Skiern. Ein entsprechender Sensor kann beispielsweise einen Sensorfühler aus schleifbarem und leitfähigem Material aufweisen, welches in Kontakt zum Schnee steht und beim Skischliff mitgeschliffen werden kann. Dies können beispielsweise alle leitfähigen Materialien sein. Die Fühlerlänge hängt von der Dicke des Skibelags und der Hitzeleitfähigkeit des Fühlers ab.

Durch Behandlung des Skis z.B. während des Wachsens, Schleifens entsteht Hitze im Ski, wobei die leitfähigen Materialien in der Regel die Hitze schneller als andere Materialien der Skibeläge ableiten, wodurch der Sensor potentiell beschädigt werden kann. Durch eine Auswahl der Materialien für die Fühler, Fühlerlänge und optional einem Puffermaterial zwischen Fühler und Sensor kann somit der Sensor geschützt werden.

Vibrationssensoren können beispielsweise genutzt werden zur Erfassung von Vibrationen in Kugellagern oder zur Erfassung des Rollwiderstandes bei straßenbasierten Sportarten wie beispielsweise Radfahren, Rollski oder Inline-Skates.

Die Erfassung des Wegrutschens (beispielsweise nach hinten aber auch seitliche Traktion) kann beispielsweise durch die Dreiachsbeschleunigungssensoren 101a oder auch durch Gyroskope, die 3D-Kamerasensoren 101c und andere Sensoren erfasst werden.

Alle genannten Sensorkomponenten können auch unabhängig voneinander als eigenständige Sensorsysteme eingesetzt werden.

Ausführungsbeispiele weisen dafür Sensorsysteme mit eigenständigen Energieeinheiten, Funkmodul und Schnittstellen auf, die jeweils eine oder mehrere der genannten Funktionalitäten bieten. Die Energiequelle 103 kann aus einer Energiemanagementeinheit, einer Ladeschnittstelle (kabelgebunden oder drahtlos per Induktion), einer Batterie, einem Akku und/oder aus einem sogenannten Energie-Harvesting bestehen. Bei vielen Sportarten entstehen aufgrund der sportlichen Betätigung Schwingungen in einen für die Energiegewinnung optimalen Frequenzbereich und sie können daher genutzt werden, um die Leistungsdauer der Batterie zu verlängern oder sogar ohne Batterie arbeiten zu können.

Gemäß weiteren Ausführungsbeispielen können die Batterien austauschbar sein oder auch aufgeladen werden, wobei für die Aufladung ein Verbindungskabel vorgesehen sein kann oder sie schnurlos beispielsweise über eine Induktionsmatte aufgeladen werden können.

Optional sind in weiteren Ausführungsbeispielen weitere Sensoren im Skikern mit drahtbasierten oder funkbasierten Verbindungen zum Hauptmodul vorgesehen. Hierfür kommen beispielsweise alle Sensorarten in Frage. Die Sensoren mit geeigneter Größe und Form können beispielsweise effizient in Karbonwaben eingeführt werden. Beispielsweise können runde oder sechs- und mehreckförmige Sensoren im Durchmesser bzw. Längenbereich zwischen 0,2 und 9 mm genutzt werden.

Optional können weitere Sensoren in anderen Skibereichen vorgesehen sein. Beispielsweise kann ein Drucksensor und/oder ein Temperatursensor und/oder ein Beschleunigungssensor in den Seitenwangen und/oder Skikanten und/oder Untergurt oder Obergurt oder Kernabschlüssen oder anderen Teilen des Skis vorgesehen sein.

Optional sind bei weiteren Ausführungsbeispielen insbesondere optisch, elastische Fasern und andere textile Drucksensoren zur Druckmessung (die auch als Druckfasern bezeichnet werden können) in dem Ski oder deren Bestandteile eingewoben oder eingebracht. Insbesondere können sie direkt in die Lauffläche des Skis eingebracht werden, beispielsweise in die sogenannte Untergurte oder Obergurte und vor allem in den aus Karbonstrukturen oder Geweben bestehenden Karbonkern.

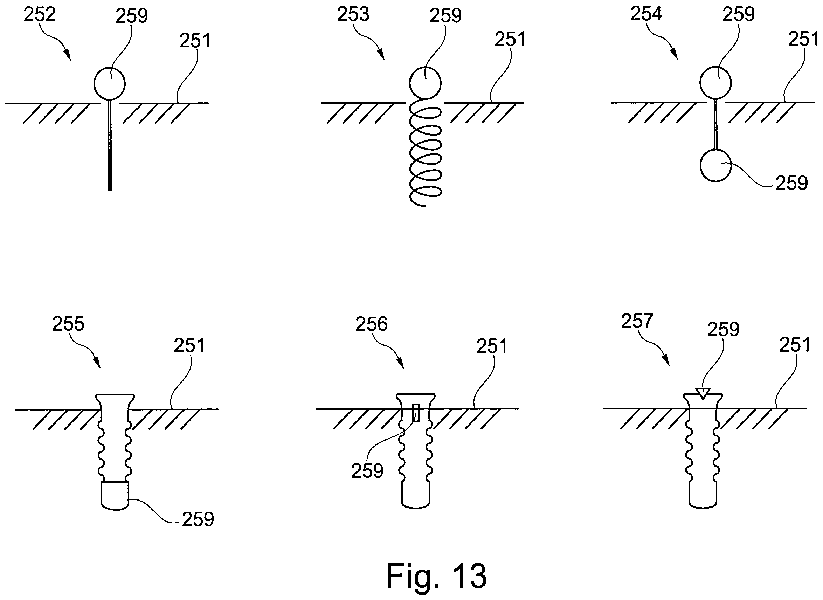

Fig. 13 zeigt ein Ausführungsbeispiel zur Anbringung des ersten oder zweiten Sensormoduls 100, 200 an einem Schuh oder Ski. Grundsätzlich funktioniert jede Form der Befestigung am Schuh, sofern der Sensor fest fixiert ist. Das folgende Verfahren ist insbesondere für die nachträgliche Integration in einem Sportartikel geeignet. Zunächst kann eine Integration des Sensors 259 in Schrauben, Nägeln oder Nadeln oder ähnlichen Befestigungsmaterialien erfolgen. Beispielsweise sind folgende Sensorintegrationen der Sensoren 259 in Skigeräte oder Schuhe 251 gezeigt: die Integration eines Sensors in einem Nadelkopf 252, die Integration des Sensors in einer spiralförmigen Nadel 253 (z.B. im Kopfteil), die Integration des Sensors 259 in ein Nadelrohr oder eine Nadelspitze 254, die Integration des Sensors 259 als Kopf an der Spitze einer Schraube 255 (d.h. innerhalb des Materials), die Integration des Sensors 259 in einer Schraube 256 (beispielsweise eingegossen oder auch nach dem Einschrauben in das Sportgerät von oben in einen Hohlraum einführbar), und die Integration des Sensors 259 in eine Kerbe der Schraube 257 nach der Verschraubung, wobei der Sensor beispielsweise eingeklebt oder befestigt wird.

Für die Qualität der Sensordaten ist es wichtig, das Sensorsystem an der richtigen Position am oder im Sportgerät zu integrieren. Hierzu können gemäß Ausführungsbeispielen die folgenden Verfahren genutzt werden. Zum Einbau von Sensorsystemen in Skier (Alpinskier oder Skilanglauf oder Snowboards) kann das folgende Verfahren genutzt werden:
Beispielsweise kann das Sensorsystem auf der Skioberseite oder der Oberflächenbeschichtung oder dem Obergurt befestigt werden. Optional ist es ebenfalls möglich, das Sensorsystem auf dem Ski durch Befestigungsmethoden wie beispielsweise Kleben, Klett-, Klick- oder Reisverschlüssen zu befestigen, wobei ebenfalls Magnete, Saug- oder Unterdruckvorrichtungen genutzt werden können. Ebenfalls ist es möglich, das Sensorsystem durch Schrauben, Schmelzen, Laminieren, Gurten mit elastischen Materialien zu befestigen. Bei anderen Ausführungsbeispielen wird das Sensorsystem auf der Skispitze aufgebracht oder aufgesteckt. Ebenso ist es möglich, die normale Skispitze durch das Bewegungsablaufüberwachungssystem in Skispitzform zu ersetzen. Bei weiteren Ausführungsbeispielen wird das Überwachungssystem innerhalb der Skispitze eingebracht oder befestigt. Weitere Ausführungsbeispiele beziehen sich auf einen Einbau in einer Skibindung oder einer Bindungsplatte, beispielsweise durch ein Einbringen in die Bindung, eine Befestigung auf, neben oder unter der Bindung oder ein Einlegen zwischen der Skibindung und/oder Bindungsplatte und/oder Ski ohne Befestigungsmaterial. Beispielsweise kann das Einbringen in die Bindung mittels Einkleben, Einschmelzen, Anfertigen eines passgenauen Hohlraumes in die Bindung oder ähnliche Methoden umgesetzt werden. Beispielsweise kann das Befestigen auf, neben oder unter der Bindung durch ein Kleben, Klett-, Klick-, Reiß-Verschlüsse, Magnete, Saugvorrichtungen oder Unterdruckvorrichtungen, durch Schrauben oder Schmelzen erfolgen.

Verfahren zum Befestigen des Sensorsystems an Schuhen wie beispielsweise Laufschuhe, Skischuhe oder Boardschuhe, Inline-Skates, können folgende Möglichkeiten umfassen:
An Laufschuhen können Drucksensoren in das Dämpfungselement oder oberhalb das Dämpfungselement angebracht sein. Dazu kann beispielsweise ein Einstechen, Einschrauben, Verkleben, ein Einweben oder ein Einschmelzen genutzt werden. Ferner ist es möglich das Sensorsystem 100, 200 mit einem Dreiachsbeschleunigungssensor an die Ferse anzubringen.

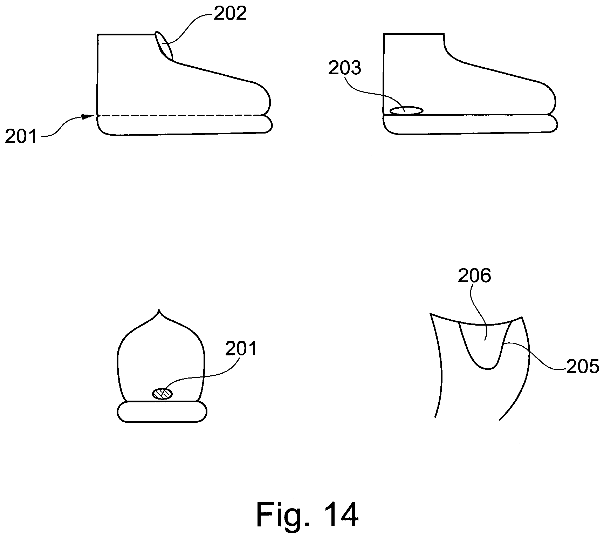

Diese Möglichkeit ist in der Fig. 14 gezeigt, wobei das Sensorsystem beim Laufschuh direkt am oberen Ende 201 des Dämpfungselementes in der Mitte der Ferse 203 angebracht sein kann. Ebenfalls möglich ist das Sensorelement ca. 1 bis 3 cm (oder 1,5 bis 2 cm) vom oberen Ende der Schuhzunge 202 anzubringen (beim Laufschuh direkt beim Knoten der Schnürung oder am Fußrücken am Übergang zum Bein). Beispielsweise kann der Sensor in die Schuhzunge eingenäht oder als wechselbares Modul in eine hierfür geschaffene Einstecktasche eingeführt werden 205, die beispielsweise durch Klett- oder Reißverschlüsse 206 verschließbar sein kann.

Da die Ferse und insbesondere die Achillessehne ein sehr empfindliches Körperteil ist und gerade hier häufig Überlastungsprobleme entstehen, sollte der Sensor bestimmte Größen nicht überschreiten. Daher wird bei weiteren Ausführungsbeispielen ein maximal 4 mm großer Dreiachsbewegungssensor 101 oder Gyroskop oder MEMS-Sensor an der Gravitationsposition angeordnet und eine drahtlose oder drahtbasierte Verbindung zum Sensorhauptmodul hergestellt. Das Hauptmodul (z.B. das zweite Sensormodul) kann sich beispielsweise an der Position 205, 202 befinden, oder an der Position 203 platziert sein. Bei weiteren Ausführungsbeispielen ist ebenfalls eine sehr dünne flexible Form auf der Schuhoberseite oder der Fersenklappe angebracht.

Im Folgenden werden Verfahren zur sensorbasierten Bewegungsablauferfassung und -analyse beschrieben.

Viele Empfehlungen von Trainern, Sport- oder Skilehrern zielen auf eine Verbesserung des Bewegungsablaufs zum theoretisch biomechanisch optimalen Bewegungsablauf ab. Diesen theoretisch optimalen Bewegungsablauf gibt es in vielen Fällen nicht bzw. ein Vergleich hiermit macht trainingsmethodisch wenig Sinn. Viele Menschen haben beispielsweise unterschiedlich lange Arme und Beine, Wirbelsäulenverkrümmungen um nur einige Beispiele zu nennen. Eine Anwendung des theoretisch optimalen Bewegungsablaufes ist daher nicht möglich und wäre wohl auch nicht leistungs- und gesundheitsfördernd.

Durch einen gezielten Einsatz des Sensorsystems zur Erfassung des Bewegungsablaufes kann der Ist-Bewegungsablauf erfasst werden. Außerdem kann durch eine Triangulation mehrerer Parameter und mehrerer Trainingseinheiten in der gleichen oder vor allem in verschiedenen Sportarten auf die individuellen Gegebenheiten des Sportlers rückgeschlossen werden, die beispielsweise die Skelettsituation oder Skelettunterschiede, Leistungsfähigkeit der verschiedenen Muskel oder Muskelpartien, Beweglichkeit der Sehnen, Gelenke, etc. umfassen. Dies eröffnet neue Möglichkeiten und Formen der Prävention und Identifikation von Fehlstellungen, Abnutzungen etc. Eine ärztliche Diagnose wird hier nicht nur mit realen Bewegungsdaten unterstützt, sondern sie kann dadurch rechtzeitig angestoßen und gegebenenfalls können unnötige Routineuntersuchungen vermieden werden.

Für die Trainings- und Schulungsmethodik bedeutet dies, dass basierend auf den optimalen theoretischen Bewegungsabläufen und den individuell erfassten Bewegungsabläufen ein individuell optimaler Bewegungsablauf erstellt wird. Trainer, Sportlehrer, Sportlern selbst und andere Fachpersonen können diesen individuell optimalen Bewegungsablauf mit ihren Erfahrungen überprüfen und in vorgegebenen Bandbreiten verändern, wobei jede Änderung auf Plausibilität geprüft werden kann.

Durch die Auswertung des Bewegungsablaufs können beispielsweise folgende Vorteile erzielt werden.

Die Sportgeräte können besser ausgewählt werden. Beispielsweise kann beim Laufen mit verschiedenen Skis gezeigt werden, welcher Ski mit welcher Vorspannung, Skibelag etc. für die individuellen Laufstil, den Trainingszustand und die körperlichen Gegebenheiten am besten geeignet ist. Die körperlichen Gegebenheiten umfassen beispielsweise die Muskelverteilung, Massen etc. Das Training mit verschiedenen Laufschuhen zeigt beispielsweise inwieweit die Schuhe für das jeweilige Pronationsverhalten geeignet sind.

Außerdem können Präventionsprogramme gegen Rückenprobleme besser gesteuert werden. So ist beispielsweise eine Rückenprävention eine zentrale Herausforderung der alternden Gesellschaft. Da die Rückenmuskulatur stark auf langausdauernde Halte- und Bewegungsarbeit ausgelegt ist, können Maximalkraft- oder Schnellkraft-orientierte Trainingsprogramme und entsprechende Erfassungen der individuellen Situationen als Ausgangsbasis für Präventionsprogramme nicht oder nur eingeschränkt sinnvoll sein. Lauf- und Skilanglaufbewegungen sind nicht nur ein optimales Präventionsprogramm, sondern sind auch perfekt geeignet, um die individuelle Situation und den Fortschritt des Rückenmuskelaufbaus zu erfassen. Rückenbeschwerden oder Fehler können somit frühzeitig erfasst werden. Beispielsweise können Fehlstellungen im Rücken, Becken etc. zu Abweichungen im Bewegungsablauf führen. Bei Krafttrainingstests im Studio oder in der Klinik fallen diese durch die Kurzfristbelastung nicht auf. Sie werden jedoch bei länger anhaltenden Messreihen automatisch ermittelt und können dadurch besser analysiert und ausgeschlossen werden. Die Ermittlung des individuellen Rückenpräventionsprogramms durch entsprechende Bewegungsabläufe, aber vor allem das Monitoring des Trainingsfortschritts und ein spielerischer Anreiz zum weiteren Fortsetzen des Trainings sind ebenfalls wichtige Komponenten.

Weitere Ausführungsbeispiele beziehen sich auf ein Verfahren zur Erfassung der Bewegung im Raum und Zeit und auf ein sogenanntes Gravitationspunkt- oder Gravitations-Kraftverfahren.

Beim Verfahren zur Erfassung der Bewegung im Raum und Zeit geht es insbesondere darum, die Bewegungsabläufe mittels Winkel zu erfassen oder aus anderen Daten zu berechnen, wobei diese Abläufe häufig eine V-Form aufweisen. Bei dem Gravitationspunkt-Verfahren geht es darum, den durch den Sportler ausgeübten Druck zu ermitteln, beispielsweise beim Laufen beim Abdrücken vom Boden zu ermitteln. Diese Kraftwirkung kann direkt oder indirekt mittels verbundener Parameter ermittelt werden (beispielsweise durch eine exakte zeitliche Koordinierung beim Abdrücken).

Weitere Ausführungsbeispiele beziehen sich auf ein Muskel- und Rückenindexverfahren, welches aufbauend auf den erfassten Sensordaten einen individuellen Zustand der verschiedenen Muskeln identifiziert und den Trainingsfortschritt durch Ausüben der Bewegungen ermittelt. Dies dient insbesondere der Rückenprävention.

Diese Verfahren zur Erfassung, Messung und Analyse der Bewegungsabläufe sollen an den folgenden Beispielen detaillierter beschrieben werden.

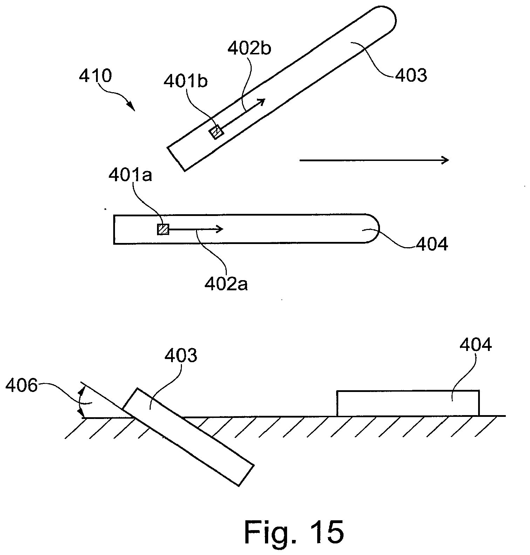

Ausführungsbeispiele für sensorbasierte Mess- und Analyseverfahren beziehen sich auf Skilanglauf, Inline-Skating, Eisschnelllauf und können wie folgt beschrieben werden:
Das V-Faktor-Verfahren misst eine optimale Gleitbewegung. Dies ist in der Fig. 15 dargestellt. Die optimale Skating-Bewegung besteht aus einem möglichst langen Gleitvorgang nach vorne und einer geringen Seitwärtsbewegung. Die Seitwärtsbewegung ist erforderlich, um den Ski leicht einzukanten und sich abzustoßen. Je stärker der seitliche Ski ausdreht wird (siehe Situation 410) oder der Ski eingekantet wird, umso mehr Energie geht in die Seitwärtsbewegung und nicht in der gewünschten Vorwärtsbewegung. Die Fig. 15 zeigt eine Draufsicht von einem Ski 403, der seitlich ausgedreht ist (siehe Position 410), um einen Abstoßimpuls zu setzen. Der Ski 404 befindet sich in einem Gleitvorgang vorwärts. Die Fig. 15 unten zeigt eine Rückansicht, wobei zu sehen ist, dass der rechte Ski 404 flach auf den Schnee aufliegt, während der linke Ski 403 seitlich gekantet und seitwärts ausgedreht ist. Die Energie geht bei dieser Bewegung durch den eingekanteten linken Ski 403 verloren. Der Sportler muss die optimale Mischung aus Abdruck und Vorwärtsbewegung finden. Dies ist je nach Schneeart und Neigung des Geländes unterschiedlich. Je größer der Anstieg bei Berghochlauf ist, umso mehr muss seitlich ausgedreht und verkantet werden, um den Halt für den Abstoßimpuls zu setzen.

Bei Ausführungsbeispielen wird das Sensorsystem (beispielsweise unter Nutzung des Dreiachsbeschleunigungssensors und Gyroskops) genutzt, um die Vorwärtsbewegung des Gleitskis und den Verlauf der Seitwärtsbewegung zu erfassen. Bei dieser Erfassung wird mit einer Startzeit, ein Geschwindigkeitsverlauf, eine Länge bis die Gleitbewegung vermindert wird bzw. in einer Seitwärtsdrehung übergeht und der Zeitpunkt des Übergangs von der Gleitbewegung in die seitwärts oder Abstoßvorbereitung gemessen. Darauf basierend wird ein Geschwindigkeitsverlauf der Bewegung bis zum Abstoßen und durch den Verlauf der nachfolgenden Rückholbewegung ohne Schneekontakt gemessen.

Bei weiteren Ausführungsbeispielen kann ein ähnliches Sensorsystem wie zuvor beschrieben genutzt werden. Optional misst ein zweites Sensorsystem (beispielsweise mit einem Dreiachsbeschleunigungs-/MEMS-Sensor) den Anstellwinkel des Skis 406 als eine Kennziffer für den optimalen Gleitvorgang.

Die Daten der Sensorsysteme können für nachfolgende Analysen gespeichert werden oder in Echtzeit in Kennziffer umgerechnet und gespeichert werden. Optional ist es ebenfalls möglich, die Daten direkt in einem Ausgabegerät an den Sportler zu geben. Das Ausgabegerät kann beispielsweise eine Sportbrille oder Sportuhr oder auch ein Kopfhörersystem sein. Der Sportler kann beispielsweise seine Abweichung von der optimalen Bewegung visualisiert bekommen. Dies kann beispielsweise durch ein Farbsystem oder Ampelsystem erfolgen, um Kategorien/Bandbreiten dem Sportler zu zeigen. Beispielsweise kann eine Brillensignalisierung einen optimalen Bereich darstellen, während eine gelbe Signalisierung eine geringe Abweichung und eine rote Signalisierung auf eine starke oder auch zu korrigierender Abweichung hindeutet. Optional ist es ebenfalls möglich, die Werte als Absolutwerte auszugeben.

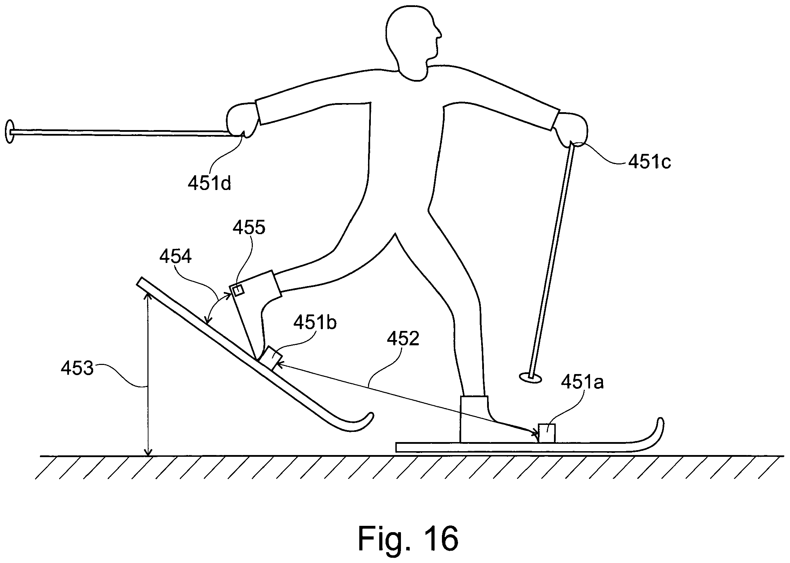

Fig. 16 zeigt ein weiteres Ausführungsbeispiel zum Einsatz des Bewegungsanalysesystems gemäß der vorliegenden Erfindung für einen Skilanglauf klassisch. Hierbei umfasst das beispielhafte Sensorsystem 451 a,b einen Dreiachsbeschleunigungssensor oder ein MEMS-Sensor oder ein Gyroskop und misst beispielsweise die Schrittlänge 452 und außerdem inwieweit der Ski nach hinten angehoben oder ausgeschwungen wird (beispielsweise durch eine Höhe 453). Optional ist es ebenfalls möglich eine Schuhhöhe 454 zu erfassen, die angibt, wie weit sich der Ski vom Schuh entfernt hat. Ein weiterer Sensor 455 ist beispielsweise in dem Schuh integriert Hierbei können insbesondere auch Responseelemente (z.B. RFID) zum Einsatz kommen.

Das Sensorsystem, wie es in der Fig. 16 gezeigt ist, kann weiter messen, ob die hintere Aufschwingbewegung gerade verläuft oder eine Seitwärtsbewegung aufweist, bzw. wann mit der Rückholbewegung des hinteren Fußes begonnen wird. Darüber hinaus ist es möglich zum Beispiel beim vorderen Bein, dem sogenannten Gleitbein, die Neigung des Skis (s.a. Fig. 15) und weitere Parameter zu messen. Dafür können beispielsweise weitere Sensoren 451 c, d in den Skistöcken und/oder Handschuhe vorgesehen sein.

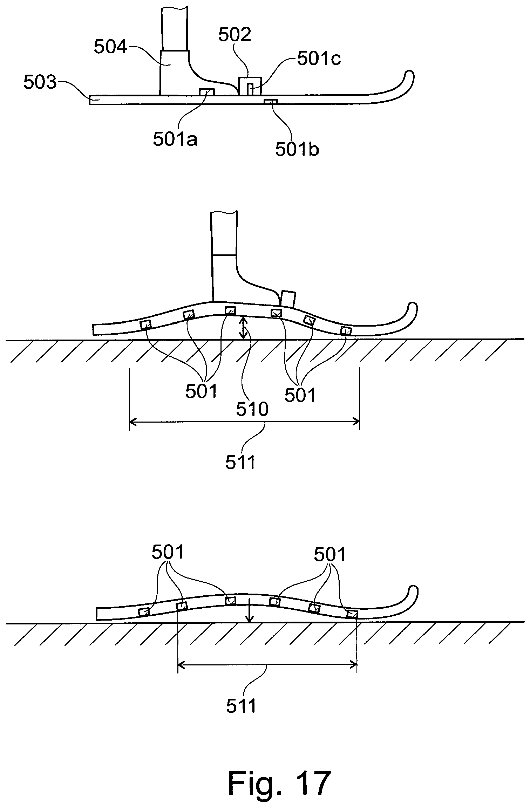

Fig. 17 veranschaulicht das Gravitationspunkt- oder Gravitations-Kraftverfahren, bei welchem die Abdrucktechnik beim Skilauf (beim Skating oder auch beim klassischen Skilauf) gemessen wird. Dazu zeigt die Fig. 17 einen Skischuh 504, der an einem Ski 503 befestigt ist. Drucksensoren 501 sind dabei im Skischuh oder unterhalb des Skis 503 oder in der Bindung 502 oder in mehreren dieser Sportgeräte angeordnet. Damit wird es möglich, den Abstoßdruck durch die Drucksensoren 501 im Ski 503, der Skibindung 502 und des Skischuhs 504 oder einem anderen Bekleidungsstück am Fuß oder Bein des Sportlers zu messen. Beispielsweise kann ebenfalls eine Socke oder einen Kompressionsstrumpf/-stutzen dafür genutzt werden. Drucksensoren sind beispielsweise Dehnungs-Messstreifen, piezoelektrische Elemente, Kraft-Momentsensoren, Garne und Stoffe zur Druckmessung mittels chemischer und physikalischer Verfahren.

Bei weiteren Ausführungsbeispielen sind entlang des Skis 503 mehrere Drucksensoren 501 angeordnet, um beispielsweise festzustellen, welcher Punkt des Skis mit welcher Kraft auf den Untergrund drückt. Damit wird es möglich, den Abdruckzeitpunkt, die Abdruckposition im Vergleich zu dem Partnerski (oder des anderen Beines/Schuhs oder des Oberkörpers) zu erfassen und die Abdrucklänge und des daraus resultierenden Vortriebs durch die Sensoren zu messen.

Die Sensoren können wiederum beispielsweise Dreiachsbeschleunigungssensoren, Gyroskope oder MEMS-Sensoren umfassen. Die Messung durch die Sensoren ermöglicht es ebenso, festzustellen, wie viel Auflagefläche im Abdruckbereich beim Abdruck den Schnee berührt oder nicht (siehe Bereich 511). Dies kann auch durch Sensoren zur Fassung des Schneekontaktes passieren. Optional kann ein Sensor, der das Ergebnis des Abdruckimpulses misst, genutzt werden, um die Bewegung des Skis in Richtung des Schnees als Geschwindigkeitskurve und tatsächlich gemessene Wegstrecke zu ermitteln (beispielsweise das Durchdrücken des leicht gewölbten Skis zu einer durchgängig flach aufliegenden Belagsfläche). Hierbei werden die durch Sensoren ermittelten Werte in Relation zur Vorspannung (entsprechend der Höhe 510 des Skis) gesetzt. Als Sensoren können wiederum beispielsweise Dreiachsbeschleunigungssensoren, MEMS-Sensoren oder Drucksensoren genutzt werden.

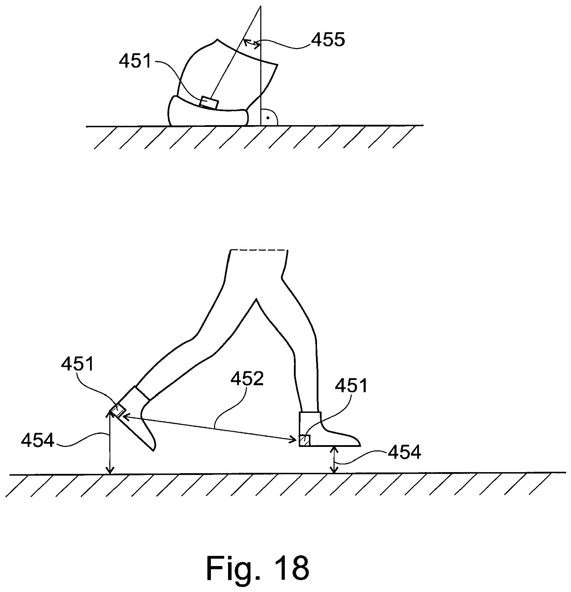

Die Vorgehensweise beim Laufen ist analog. Fig. 18 zeigt die Anwendung für eine Laufbewegung, bei der ein Läufer an seinen Laufschuhen Sensoren 451 aufweist, wobei Sensoren 451 wiederum am rechten und/oder am linken Fuß angeordnet sind. Daher ist ein Sensorsystem 451, welches wiederum beispielsweise Dreiachsbeschleunigungssensoren, MEMS-Sensoren und Gyroskope aufweisen kann, ausgebildet, um nicht nur eine exakte Schrittlänge 452 links und rechts separat zu messen, sondern auch die Ursachen hierfür zu ermitteln. Eine Schlenkbewegung des einen Fußes in der Luft kann zu einem verkürzten Schritt des anderen Beines/Fußes führen, obwohl der Abdruckimpuls richtig und kräftig war. Darüber hinaus kann das Sensorsystem messen, wie weit der Fuß nach vorne und nach hinten gebracht wird. Ebenso kann eine sich ändernde Flughöhe 454 ausgeben werden. Optional ist es ebenfalls möglich, festzustellen, ob die Fuß- oder Beinbewegung gerade verläuft oder eine Schlenkbewegung ausgeführt wird. Ebenso möglich ist es festzustellen, welche horizontale Neigung der Fuß beim Aufsetzen hat.

Beispielsweise zeigt die Fig. 18, dass der Fuß in einem Winkel 455 aufsetzt, was durch eine des Sensorelements 451 festgestellt werden kann, da die Beschleunigung nicht senkrecht nach unten wirkt sondern leicht seitlich versetzt sein wird. Daher kann das Sensorsystem feststellen, welche Neigung jeweils beim Abrollen des Fußes und beim Abdrücken vorliegt. Diese Informationen liefern nicht nur für die Lauftechnik wertvolle Verbesserungshinweise, sondern bieten auch Kriterien für eine Schuhauswahl, die dahingehend erfolgen kann, inwieweit ein bestimmter Schuh ein bestimmtes Abrollverhalten toleriert oder unterstützt.

Bei dem Gravitationspunkt-Verfahren sind beispielsweise verschiedene Drucksensoren in die Dämpfungssohle oder -schicht des Laufschuhs angeordnet. Die Drucksensoren können auch in der Schuhsole selbst oder in einer Einlegesohle vorhanden sein, so dass sie die beim Aufsetzen, Abrollen und Abdrücken des Fußes auftretenden Kräfte ermitteln können. Optional ist es ebenfalls möglich, die Kräfte mittels der zuvor beschriebenen Verfahren durch andere Sensoren (beispielsweise Dreiachsbeschleunigungssensoren) zu ermitteln.

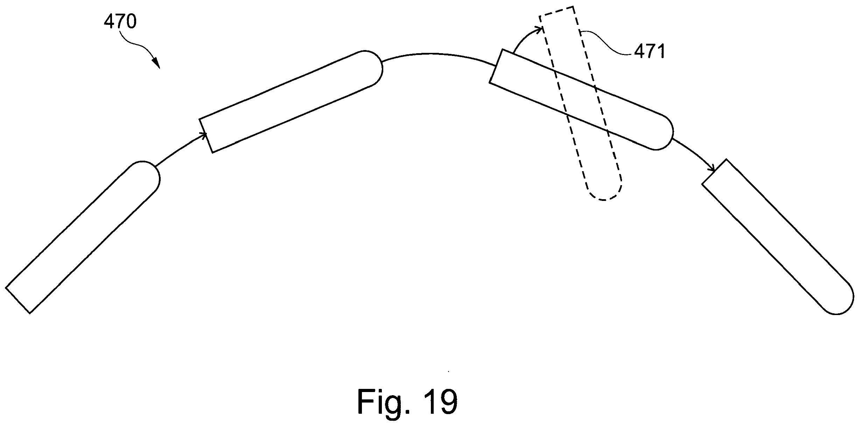

Fig. 19 zeigt eine Anwendung des Verfahrens für Ski alpin oder Snowboard oder Sommerboards, die das V-Faktor-Verfahren nutzen können. Dazu werden Daten verschiedener Sensoren aus Mehrachsbeschleunigungssensoren, MEMS, etc. erfasst und analysiert. Damit kann ein Kurvenverhalten 470 analysiert werden und beispielsweise ein Abdriften 471 von einer Optimallinie berechnet werden. Weiterhin ist es ebenfalls möglich, einen Neigungswinkel der Skischuhe zu ermitteln, um eine richtige Gewichtsverlagerung indirekt berechnen zu können.

Bei dem Gravitationspunkt-Verfahren in Bezug auf Ski alpin ist es wiederum möglich eine Druckverteilung auf den Ski oder den Schuh durch die Drucksensoren zu ermitteln und somit eine richtige Gewichtsverlagerung zu ermöglichen (z.B. durch eine Rückkopplung an den Nutzer).

Weitere Ausführungsbeispiele der vorliegenden Erfindung beziehen sich auch auf die folgenden Beispiele:
Ein Bewegungsablauf-Überwachungssystem für einen Ski, welches folgende Merkmale aufweist: einen oder mehrere Sensoren, eine Mikrokontrollereinheit (MCU), eine Energiequelle und optional eine Speichereinheit und/oder ein Funkmodul. Die Sensoren können optional Sensoren zur exakten Erfassung der Bewegung des Skis umfassen. Beispielsweise können die Sensoren einen Mehrachs-Bewegungssensor oder einen Beschleunigungssensor, MEMS-Magnetfeldsensoren oder Gyroskope umfassen.

Bei einem weiteren Beispiel umfasst die Energiequelle eine Batterie und/oder einen Akkumulator und/oder einen sogenannten Energie-Harvester, der ausgebildet ist, die bei vielen Sportgeräten entstehende durch die sportliche Betätigung verursachten Schwingungen für Energieerzeugung in einem optimalen Frequenzbereich zu nutzen.

Gemäß einem weiteren Ausführungsbeispiel ist das Überwachungssystem ausgebildet, um die Batterie über ein Verbindungskabel oder schnurlos, insbesondere über eine Induktionsmatte aufzuladen.

Bei weiteren Ausführungsbeispielen sind weitere Sensoren im Skikern mit drahtbasierten oder funkbasierten Verbindungen zu einem Hauptmodul integriert. Hierfür kommen alle Sensortypen in Frage. Beispielsweise weisen die dort integrierten Sensoren eine geeignete Größe und Form auf, so dass sie effizient in die Karbonwaben eingeführt werden können. Beispielsweise können sie eine runde oder sechs- oder mehreckige Form aufweisen und einen Durchmesser bzw. einen Längenbereich zwischen 0,2 und 9 mm umfassen.

Bei weiteren Ausführungsbeispielen sind weitere optionale Sensoren in anderen Bereichen des Skis angeordnet. Dies könne beispielsweise ein Drucksensor sein und/oder ein Temperatursensor und/oder Beschleunigungssensoren, die beispielsweise in den Seitenwangen, Skikanten, Untergurt oder Obergurt und Kernabschlüssen angeordnet sein können.

Ausführungsbeispiele der vorliegenden Erfindung beziehen sich ebenfalls auf einen Einbau und eine Befestigung des Bewegungsablaufs-Überwachungssystems in einem Ski und/oder Snowboard, wobei dazu folgende Verfahren eingesetzt werden können:
Gemäß Ausführungsbeispielen kann der Einbau auf der Skioberseite oder der Oberflächenbeschichtung oder dem Obergurt erfolgen.

Gemäß weiteren Ausführungsbeispielen kann eine Befestigung auf dem Ski durch Befestigungsmethoden wie beispielsweise Kleben, einen Klettverschluss, einen Klickverschluss, einen Reißverschluss, mit Magneten, eine Saug- bzw. Unterdruckvorrichtung, Schrauben, Schmelzen, Laminieren, Gurten mit elastischen Materialien erfolgen.

Gemäß weiteren Ausführungsbeispielen erfolgt das Aufbringen oder Aufstecken auf eine Skispitze, ein Ersetzen der normalen Skispitze durch ein Bewegungsablaufs-Überwachungssystem in Skispitzform, ein Einbringen oder Befestigen innerhalb der Skispitze.

Gemäß weiteren Ausführungsbeispielen erfolgt der Einbau in die Skibindung oder in eine Bindungsplatte des Skis. Dies kann optional durch ein Einbringen in die Bindung erfolgen, die beispielsweise ein Einkleben, ein Schmelzen, Anfertigen eines passgenauen Hohlraumes in der Bindung umfassen kann. Eine Befestigung auf, neben oder unter der Bindung kann beispielsweise ein Kleben, einen Klettverschluss, einen Klickverschluss, einen Reißverschluss, Magnete, Saugvorrichtung oder Unterdruckvorrichtung, ein Schrauben oder Schmelzen umfassen. Das Einlegen zwischen der Skibindung und/oder Bindungsplatten und/oder dem Ski kann auch ohne Befestigungsmaterial erfolgen.

Gemäß weiteren Ausführungsbeispielen sind Sensoren zur Erfassung der Gleitfähigkeit von Sportgeräten zum Gleiten auf Wasser in gefrorenem Zustand, insbesondere Schnee und Eis, vorgesehen. Diese Gleitfähigkeitssensoren sind insbesondere für Skier im Alpinen, Freestyle, Langlauf, Snowboard und Schlittschuhe vorgesehen, und erfassen physikalisch-chemische Parameter (beispielsweise über eine elektrische Leitfähigkeitsmessung oder eine Spektralanalyse). Die Sensoren zur Erfassung der Gleitfähigkeit sind weiter ausgebildet, um den Gleitvorgang der Sportgeräte auf Schnee und Eis und die damit verbunden dünne Wasserschicht zwischen der Gleitfläche des Sportgerätes (z.B. Skibelag oder Kufe) und dem Schnee zu nutzen und durch ein Erfassen der physikalisch chemischen Parametern die Gleitfähigkeit des Sportgerätes zu bestimmen.

Gemäß weiterer Ausführungsbeispiele erfolgt eine direkte Sensormessung in der Lauffläche des Skis, wobei ein Sensorfühler aus schleifbarem, leitfähigem Material, welches Kontakt zum Schnee hat und beim Skischliff mitgeschliffen wird, besteht. Dies können alle leitfähigen Materialien sein. Die Fühlerlänge hängt von der Dicke des Skibelags und der Hitzeleitfähigkeit des Fühlers ab. Ausführungsbeispiele berücksichtigen hierbei, dass bei der Behandlung des Skis beispielsweise durch ein Wachsen oder Schleifen des Skis Hitze entsteht und leitfähige Materialien in der Regel die Hitze schneller als Materialien der Skibeläge leiten, wodurch der Sensor beschädigt werden könnte. Daher weisen Ausführungsbeispiele insbesondere ein Material für Fühler auf, oder die Fühlerlänge ist derart gewählt. Optional ist das Material als ein Puffermaterial zwischen dem Fühler und dem Sensor angeordnet, um den Fühler entsprechend zu schützen.

Gemäß weiteren Ausführungsbeispiele erfolgt eine Messung der Abdrucktechnik beim Skilaufen (beispielsweise beim Skaten oder beim klassischen Skilaufen) durch folgende Schritte: Messung eines Abstoßdruckes durch einen oder mehrere Drucksensoren; Messung eines Abdruckzeitpunktes; und Messung wie viel Lauffläche im Abdruckbereich beim Abdruck mit dem Schnee in Kontakt sind. Bei weiteren Ausführungsbeispielen ist der Drucksensor im Ski, in der Skibindung, im Skischuh oder einem Bekleidungsstück für den Fuß oder das Bein angeordnet. Beispielsweise kann der Drucksensor auch in Socken oder Kompressionstulpen angeordnet sein. Die Drucksensoren können beispielsweise Dehnungsmessstreifen, piezoelektronische Elemente, Kraftmomentsensoren, Garne und Stoffe zur Druckmessung mittels chemischer und physikalischer Verfahren umfassen.

Optional ist es ebenfalls möglich, dass bei der Messung des Abdruckzeitpunktes, die Abdruckposition im Vergleich zum Partnerski und/oder des anderen Beines/Schuhs und/oder des Oberkörpers ermittelt werden. Ebenfalls ist es möglich, die Abdrucklänge und des daraus resultierenden Vortriebs durch die Sensoren zu erfassen, wobei die Sensoren insbesondere eine Dreiachsbeschleunigungssystem, Gyroskope und MEMS-Sensoren umfassen können.

Optional werden bei der Messung der Lauffläche die durch die Sensoren ermittelten Werte in Bezug zu einer Vorspannung (Spannung und Höhe des Skis) gesetzt. Auch für diesen Schritt kommen beispielsweise Dreiachsbeschleunigungssensoren, MEMS-Sensoren und Drucksensoren zum Einsatz.

Weitere Ausführungsbeispiele beziehen sich auf ein Verfahren zur Ermittlung des individuell optimierten Soll-Bewegungsablaufes. Beispielsweise kann auf Basis der durch die genannten Sensorsysteme ermittelten Ist-Bewegungsabläufe weitere biomechanische und medizinisch ermittelte Daten im Vergleich zum optimalen Bewegungsablauf der Sportart gesetzt werden. Mit Veränderungen/Beeinflussungen durch Eingabe von einem Trainer, Sportlehrer, Sportler selbst und anderen Fachpersonen sind weitere Einflussmöglichkeiten (wie beispielsweise eine Plausibilitätsprüfung) gegeben.

Bei weiteren Ausführungsbeispielen erfolgt die Messung, Analyse, Monitoring des Bewegungsablaufen und der umgebenden Objekte mittels 3D-Sensoren oder 3D-Kameras in den Sportgeräten (beispielsweise Ski und Boards). Die 3D-Sensoren/Kameras erfassen nicht nur die eigene Bewegung (wie beispielsweise eine Veränderung zur Umgebung wie Geschwindigkeit, indirekte Berechnung des Abstoßdrucks durch Triangulation mit den Umgebungsdaten), sondern Erfassen ebenfalls die Bewegung der anderen Körperteile und Sportgeräte (beispielsweise ein Erfassen und Auswerten des anderen Beines).

Weitere Ausführungsbeispiele beziehen sich auf ein Verfahren und Methoden zur Erfassung des Widerstandes bei dem durch den Sport erzielten Vortrieb und andere Ergebnis mindernde Faktoren. Die hierbei erfassten Werte umfassen beispielsweise (diese Aufzählung ist nicht ausschließend anzusehen): eine Messung des Windwiderstandes für alle stark vortriebsorientierten Freiluftsportarten (z.B. Laufen, Ski, Skilanglauf, Radfahren), durch entsprechende Sensoren und miniaturisierte Messgeräte. Weiterhin ist es möglich, die Gleitfähigkeit der Sportgeräte beim Gleiten auf Wasser in gefrorenem Zustand (Schnee und Eis) zu messen. Insbesondere, aber nicht ausschließlich können Ski (alpin, Freestyle, Langlauf), Snowboards, Schlittschuhe durch Erfassen von physikalisch-chemischen Parametern (z.B. die elektrische Leitfähigkeit oder Spektralanalyse) gemessen werden.

Beispielsweise kann das Verfahren zumindest einige der folgenden Details umfassen: Bestimmen der Gleitfähigkeit der Sportgeräte durch Erfassen von physikalisch-chemischen Parametern; Einsatz von schleifbaren/abreibbaren und die Sensorinformationen transportierbaren Verbindungsmaterialien (Fühler), die zwischen dem Skibelag und dem Einzelsensor oder dem Sensorhauptmodul angeordnet sein können. Die Fühlerlänge berechnet sich beispielsweise durch die Dicke des Skibelags, des Skis und der Hitzefähigkeit des Fühlers. Erfassung von Vibrationen im Kugellager zur Erfassung eines Rollwiderstandes bei straßenbasierten Sportarten wie beispielsweise Radfahren, Rollski und Inline-Skates. Bei weiteren Ausführungsbeispielen erfolgt eine Erfassung des Wegrutschens (Traktion) nach hinten aber auch seitlich durch ein Dreiachsenbeschleunigungssensor, Gyroskop oder eine 3D-Kamera-Sensoren.

Mit den Fig. 20 bis 23 wird im Folgenden das Gravitationsdruckpunkt-Verfahren im Detail veranschaulicht. Das Gravitationsdruckpunkt-Verfahren führt eine indirekte Berechnung oder Abschätzung einer Kraftwirkung (Höhe der Kraftwirkung und Richtung bzw. Richtungsverlauf) mit Beschleunigungssensoren durch (wobei der Einsatz eines Gyroskops ergänzend sinnvoll bzw. erforderlich sein kann). Die Beschleunigungssensoren ermitteln die Beschleunigungsdaten bei der Kompression von Materialien, beim Abdämpfen einer Aufprall-Bewegung oder beim Durchdrücken bei einer Abdruck-Bewegung und setzen sie in Relation zum Härtegrad oder den Kompressionseigenschaften der Materialien.

Es ist besonders für Anwendungen geeignet, bei denen es genügt die Kraftwirkung nur in Bandbreiten oder Kategorien zu ermitteln, d.h. wo nicht zwingenderweise exakte Messungen erforderlich sind.

Insbesondere wenn andere Mess-Verfahren oder Sensoren (z.B. Dehnungsmessstreifen) nicht eingesetzt werden können, ist der Einsatz des Gravitationsdruckpunkt-Verfahrens sinnvoll. Zum Beispiel, wenn: (1) die Lebensdauer bzw. Haltbarkeit von Druck-Sensoren wie beispielsweise Dehnungsmesstreifen oder kapazitiven Elementen in Schuh-Sohlen bei der Nutzung nicht ausreichend gegeben oder (2) der Einsatz zu teuer oder (3) Produktionstechnisch zu aufwendig bzw. kompliziert ist oder (4) die Sensoren nicht nachträglich eingebaut werden können. Es ist insbesondere für den Einsatz bei Sportaktivitäten wie Skilanglauf oder Laufen bzw. Laufschuhe geeignet.

Wenn folgende Voraussetzungen vorliegen ist der Einsatz des Gravitationsdruckpunkt-Verfahrens besonders geeignet: (a) Zwischen dem Körper (des Athleten oder Sportlers), für den die Kraft bestimmt werden soll, und dem Untergrund befindet sich ein verformbares Material, dessen Härte bzw. Verformbarkeitskurve oder Kompressionsverlauf bekannt ist oder bestimmt werden kann. (b) Ein fester Untergrund angenommen werden kann, so dass die eingesetzte Kraft nur das Material verformt und nicht vom Untergrund zusätzlich abgeleitet wird. Bei weichem Untergrund - wie weichem Schnee, Waldboden etc. - kann es zu einer Ableitung der Kompression durch den weichen Untergrund kommen. Allerdings kann auch diese Kompression des Untergrundes von den Beschleunigungssensoren erfasst werden. Da sich aber diese Kompression in Höhe und Verlauf von der Kompression des Sportgeräts (Laufschuh, Ski) unterscheidet, kann die Kompression des Untergrundes identifiziert und entsprechend korrigierend bei den Berechnungen der Kraft berücksichtigt werden. Zusätzlich können diese Störeffekte des Untergrundes durch geeignete statistische Verfahren (MittelwertBildung etc.) eliminiert, gefiltert oder minimiert werden.

Zur indirekten Berechnung oder Abschätzung der Kraft beim Abdruck ist die Ermittlung des Gravitationsdruckpunkts sinnvoll, um eine genauere indirekte Berechnung oder Abschätzung der Kraft zu ermöglichen. Um den Gravitationsdruckpunkt zu ermitteln, wird durch einen Test (eine Art Eichung) ermittelt oder berechnet, inwieweit sich das verformbare Material durchdrückt, wenn der Körper in Ruhe darauf steht, d.h. nur das Gewicht des Körpers und damit die Gravitationskraft auf das verformbare Material wirken. Somit wird der Druckpunkt der Gravitationskraft ermittelt, d.h. ein Null-Punkt, wenn der Athlet selbst keine Kraft ausübt und nur die Gravitationskraft wirkt und das Material komprimiert. Von dieser Betrachtung leitet sich der Name des Verfahrens ab.

Wenn der Körper eine Kraft ausübt und Arbeitsleistung erbringt, wird das verformbare Material weiter zusammen bzw. nach unten gedrückt. Ausgehend von diesem Gravitationsdruck- bzw. Null-Punkt kann das weitere Zusammendrücken durch einen Beschleunigungssensor ermittelt werden, d.h. die Beschleunigungen und Richtungen im 3-Dimensionalen Raum können erfasst werden. Durch Kenntnis des Härtegrades des Materials bzw. der Kompressionsverläufe, kann entsprechend die Kraft und deren Richtung berechnet werden.

Dieser Sachverhalt ist am Beispiel "Laufen" in der Fig. 20 dargestellt, wobei insbesondere die Dämpfung beim Aufsetzen des Laufschuhs am Boden berücksichtigt wird (siehe Fig. 20C):
Fig. 20A: der Fuß befindet sich in der Luft und es wirkt keine Kraft auf die Dämpfungssohle, d.h. der Fuß weist noch keinen Bodenkontakt auf, wobei die Sohle des Schuhes eine Dicke A aufweist.
Fig. 20B: der Läufer steht mit beiden Beinen in Ruhestellung auf den Schuhen bzw. den beiden Dämpfungssohlen und lediglich die Schwerkraft G des Sportlers wirkt auf den Fuß und die Sohle. Infolge der Elastizität oder Kompressibilität der Sohle und des Gewichts des Sportlers verringert sich die Dicke der Sohle auf einen Wert B. Das Gewicht des Läufers mit Kleidung etc. ist bekannt (kann vorher eingegeben werden) und die Kompression kann gemessen werden (je nach Härte der Dämpfungssohle wird dies sehr unterschiedlich ausfallen; dieser Gravitationsdruckpunkt kann zum Beispiel auch maschinell für bestimmte Gewichtsklassen z.B. 50-60 kg, 61-70 kg, 71 - 80 kg etc. ermittelt werden). Unterhalb der Fig. 20B ist die weitere Deformation (von Sohlendicke A zu einer Sohlendicke B) infolge der Schwerkraft (schematisch vergrößert) dargestellt. Ein weiterer Test ist das wechselseitige, einseitige Belasten des linken und rechten Beines. D.h. der Sportler belastet zunächst mit beiden Beinen gleichmäßig das oder die Sportgeräte. Dann belastet er nur ein Bein und dann das andere. Aus diesem wechselseitigen Belasten können (ergänzende) Informationen über die Kompressionseigenschaften der Materialien ermittelt oder überprüft werden. Hierfür wird das dieser Erfindung zugrunde liegende tragbare Bewegungsanalysesystem mit zwei integrierten und in Echtzeit Informationen austauschenden Sensorsystemen genutzt.
Fig. 20C: der Läufer landet beim Laufen aus entsprechender Sprung-/Laufhöhe und mit entsprechender Laufgeschwindigkeit auf dem Boden. Als Folge wirkt die Schwerkraft G und die Kraft F, die vom Sportler aufgebracht wird, auf die Sohle, die daher wesentlich stärker durchgedrückt wird als bei Fig. 20B. Der resultierende Wert C ist kleiner als der Wert B (siehe vergrößerte Darstellung). Diese weitere Verringerung der Sohlendicke ist unterhalb der Fig. 20C wiederum vergrößert dargestellt. Beispielsweise kann das 2,5 - 3,5-fache des Körpergewichts des Läufers auf die Schuhsohle einwirken. Die Kraftwirkung hängt von der Laufgeschwindigkeit und auch dem Laufstil des Läufers (z.B. Sprung-/Laufhöhe, die von der Abdruck-Bewegung des Läufers abhängt) ab. Zur Berechnung der Kraftwirkung wird die Differenz zwischen B und C herangezogen und mit Hilfe des bekannten Härtegrades des Materials bzw. der Kompressionsverläufe die Kraftwirkung berechnet. Darüber hinaus können aber durch die Auswertung der Beschleunigungsverläufe zwischen B und C in Relation zu den Kompressionsverläufen die Berechnungen der Kraftwirkung validiert oder deren Genauigkeit verbessert werden.

Die Fig. 20B definiert somit den Gravitationsdruckpunkt, das heißt jenen Punkt, bei dem während einer Bewegung lediglich die Gravitationskraft des Sportlers auf das Sportgerät wirkt. Bei Kenntnis der Kompressionseigenschaften des Materials kann auf die Ermittlung des Gravitationsdruckpunkts verzichtet werden. Um die Kompressionseigenschaften zu kontrollieren, kann aber in vielen Fällen ein Test sinnvoll oder sogar notwendig sein. Insbesondere um Veränderungen der Kompressionseigenschaften bzw. des Härtegrades im Zeitablauf oder durch Nutzung bzw. Abnutzung zu erfassen und zu kontrollieren (z.B. bei Laufschuhen).

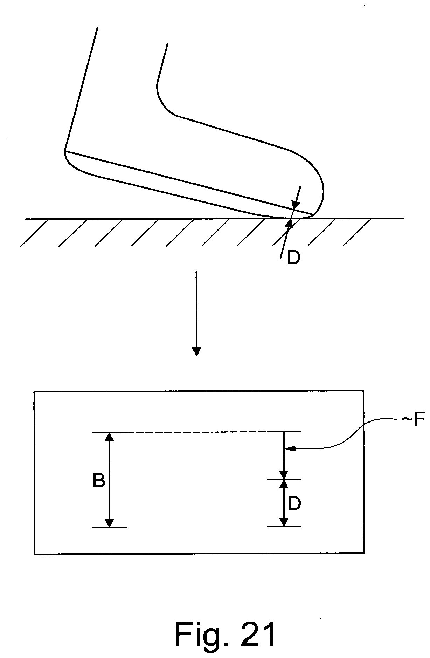

Fig. 21 zeigt entsprechend das Abdrücken des Läufers vom Boden. Durch die Kraft F des Abdrucks wird die Schuhsohle zusammengedrückt, d.h. die Sohlendicke B, die den Gravitationsdruckpunkt definiert (siehe Fig. 20B), wird infolge des Abdrückens weiter verringert auf einen Wert D. Die Wegstrecke des Zusammendrückens, aber insbesondere die damit verbundene Beschleunigungsdaten (der Kurvenverlauf), ermöglichen ein indirektes Berechnen der vom Läufer eingesetzten Kraft F (insbesondere auch deren Richtung).

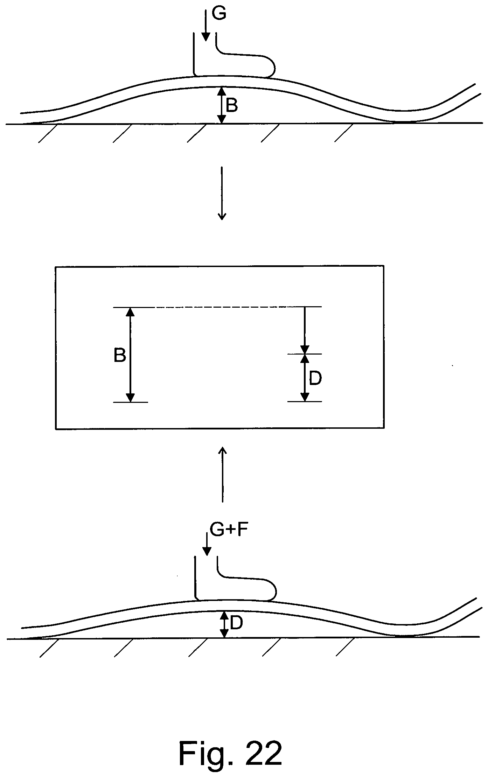

Figur 22 zeigt das Beispiel eines Langläufers beim Abdrücken des Skis bei der Skating-Technik. In der Fig. 22 oben ist zunächst wieder der Gravitationsdruckpunkt dargestellt, der jenem Abstand B entspricht, wo auf den Ski lediglich die Gravitationsschwerkraft G des Sportlers wirkt und somit einen bestimmten Punkt innerhalb der Bewegung des Sportlers darstellt. Dadurch werden die Härte bzw. Kompressionseigenschaften der Ski überprüft oder ermittelt.

In diesem Anwendungsfall ist es aber wichtig, die Neigung zu berücksichtigen. Beim Skilanglaufen in der Skating-Technik stößt der Sportler sich beispielsweise seitlich ab, so dass beim Abdruck oder Abstoss nicht oder nicht das volle Gewicht des Sportlers auf dem Ski lastet und der Ski nicht durch das Gewicht des Sportlers bzw. die Gravitationskraft bis zum Gravitationsdruckpunkt (B) durchgedrückt wird. Je nach Neigung des Skis kann dann zum Beispiel der Punkt A für die Berechnung der vom Sportler eingesetzten Kraft relevant sein (oder Punkte zwischen A und B; dies kann durch die Neigung beim abstoßenden Ski ermittelt werden). Die Neigung beim Skilauf kann beispielsweise durch ein (Magnet-) Gyroskop ermittelt oder kontrolliert werden.

Daher wird beim Erfassen der ersten und/oder zweiten Beschleunigungswerte insbesondere die Richtung und der Neigungswinkel der Extremität(en) und des Skis mit erfasst. Außerdem vergleicht die erste und/oder die zweite Verarbeitungseinheit die Richtung und den Neigungswinkel der erfassten Beschleunigungswerte von dem ersten und/oder zweiten Beschleunigungssensor mit dem Bewegungsprofil.

In der Fig. 22 unten ist weiter der Fall gezeigt, wo der Abstand B sich weiter auf einen Abstand D verringert hat (siehe vergrößerte Darstellung in der Mitte; wie oben erläutert kann der hier gezeigte Wert B aber dem Wert A entsprechen, wenn der abstoßende Ski stark geneigt ist), der dem Abstand D entspricht, wie er beispielsweise in der Fig. 21 für ein Abrollen beim Laufen dargestellt ist. Somit ist die Verringerung des Abstandes von dem Wert B auf den Wert D ein Maß für den Abdruck oder Abstoßen (der Kraft F) des Läufers beim Skilaufen in der Skating-Technik

Ein wichtiger Vorteil der indirekten Messung der Kraft beim Aufsetzen des Schuhs oder des Abdrucks beim Laufen oder Skilanglaufens gegenüber anderen Methoden (z.B. Einlegesohlen mit kapazitiven Elementen zur Druck- bzw. Kraftmessung) ist, dass nicht nur die Beschleunigung bzw. das Abbremsen und damit die Kraft-Wirkung ermittelt, sondern auch die genaue Kraft-Richtung gemessen wird. Wird zum Beispiel die Kraft beim Abbremsen des Laufschuhs beim Aufsetzen seitlich abgeleitet, können sogenannte Über-Pronationen oder Supinationen mit negativen gesundheitlichen Folgen begünstigt oder verursacht werden. Durch den Einsatz anderer Dämpfungsmaterialien oder einer anderen Zusammensetzung verschiedener Dämpfungsmaterialien kann dies dann verhindert oder zumindest vermindert werden. Dies stellt eine wichtige Informationen für die Neugestaltung von Laufschuhen oder die Auswahl geeigneter Schuhe etc. dar.

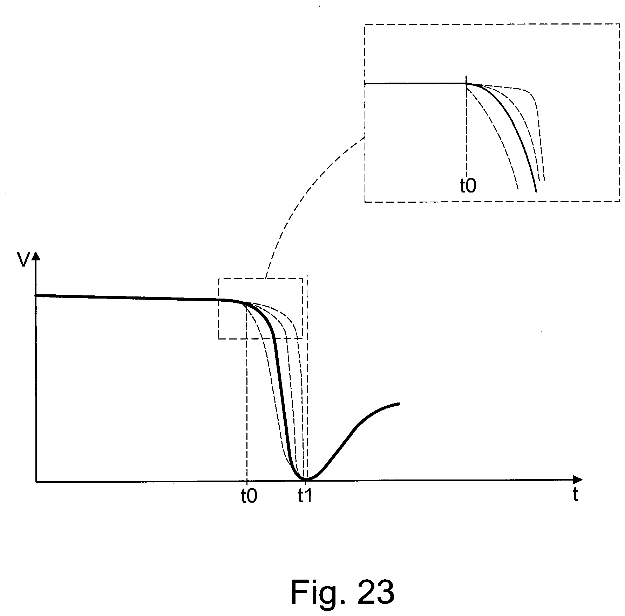

Die Fig. 23 stellt die funktionale Abhängigkeit des Geschwindigkeitsbetrages v des Sportlers (oder des Sportgerätes) mit der Zeit t dar, wie sie beispielsweise beim Laufen durch die Beschleunigungssensoren erfasst werden kann. Es versteht sich, dass eine erreichte Geschwindigkeit oder eine Geschwindigkeitsverlauf durch fortlaufende Beschleunigungsmessungen (Aufsummation) ermittelt werden kann. In der Fig. 23 bewegt sich der Schuh in Richtung Boden und wird beim Bodenkontakt zur Zeit t0 durch das Dämpfelement schnell abgebremst, d.h. die Geschwindigkeit wird schnell verringert bis sie zum Zeitpunkt t1 Null ist und anschließend wieder ansteigt. Im vergrößerten Bild ist skizziert, dass der Kurvenverlauf beim Abbremsen sich stark unterscheiden kann. Dies ist von der einwirkenden Kraft und dem Kompressionsverlauf des Materials abhängig. Da der Kompressionsverlauf des Materials ermittelt werden kann oder bekannt ist, kann durch die Ermittlung des Kurvenverlaufs beim Abbremsen die Kraftwirkung ermittelt werden. Es ist zu beachten, dass dies eine stark vereinfachte Skizze ist, da die Bewegung im 3-Dimensionalen Raum stattfindet und die Sensor-Daten bzgl. x-, y-, und z-Achse erfasst werden. Trotzdem kann aus dem Verlauf, wie schnell die Geschwindigkeit v vom Zeitpunkt t0 bis zum Zeitpunkt t1 auf einen Wert Null (oder nahe Null) sinkt und des bekannten Elastizitätswertes der Sportausrüstung verbunden mit dem Gewicht des Sportlers ermittelt werden, mit welcher Krafteinwirkung der Sportler auf den Boden aufsetzt. Beispiele für unterschiedliche Verläufe sind in der Vergrößerung durch die gestrichelten Linien dargestellt. Es ist zu beachten, dass die Kurven den Absolutbetrag der Geschwindigkeit v darstellen und nicht die Bewegungsumkehr berücksichtigen, die nach einem Aufsetzen, Abrollen und durch ein Abstoßen eines beispielhaften Läufers beim Laufen beim Bodenkontakt entsteht.

Außerdem ist zu beachten, dass die gezeigten Kurven von der Temperatur und der Elastizität des Materials der Sportausrüstung (beispielsweise der Schuhe oder des Skiers) abhängen. Für (nahezu) konstante Temperaturen und der Elastizität sind jedoch die einzelnen Kurven der Fig. 23 ein direkter Hinweis darauf, mit welcher Krafteinwirkung er auf den Boden aufsetzt. Diese Werte/Kurven können durch die Module erfasst und zur integrierten Auswertung genutzt werden. Zum Ausschließen von Temperaturänderungen oder Elastizitätsänderungen können weitere Sensoren in der Sportausrüstung vorgesehen sein. Z.B. kann die Temperatur durch einen Temperatursensor gemessen werden oder Sensoren können die Deformierbarkeit der Sportausrüstung messen bzw. kontrollieren (die Ermittlung der Kompressionsverläufe der Materialien bei verschiedenen Temperaturbereichen und entsprechende Berücksichtigung bei der Berechnung ist eine weitere Alternative). Um die Werte korrekt interpretieren zu können, wird gemäß Ausführungsbeispiele sowohl das Gewicht des Sportlers als auch Materialeigenschaften (z.B. Kompressions- oder Elastizitätsfähigkeit) der Sportausrüstung beispielsweise in der beispielhafte Speichereinheit 204 erfasst. Diese Daten können beispielsweise durch den Sportler selbst eingegeben werden. Die Kompressionsfähigkeit kann aber auch voreingestellt oder durch das Sensorsystem bei Tests ermittelt werden.

Außerdem kann bei weiteren Ausführungsbeispielen ein weiterer Eich- und Kalibrierungsprozess vorgesehen sein, der zunächst die Beschleunigungssensoren bzgl. Erdbeschleunigung, deren Richtung, etc. prüft, so dass durch eine Subtraktion der Schwerkraftwirkung (unter Nutzung des Gewichts des Sportlers) aus den gemessenen Beschleunigungswerten die Kraftwirkung F, die der Sportler aufwendet, ermittelt werden kann.

Durch das dieser Erfindung zugrundeliegende integrierte System mit zwei Sensorsystemen, kann der durch das Gravitationsdruckpunkt-Verfahren ermittelten Krafteinsatz eines Sportlers auf einem Fuß in direkten Bezug zur Wirkung auf dem anderen Fuß und dem gesamten Bewegungsablauf gesetzt werden. Beim Laufen drückt sich der Sportler auf einem Fuß ab (Ermittlung des Krafteinsatzes durch das Gravitationsdruckpunkt-Verfahren) und dadurch wird der Körper - und dadurch auch der zweite Fuß mit dem daran befindlichen zweiten Sensor-System - beschleunigt. Diese Beschleunigung des zweiten Fußes ist das Ergebnis des Abdrucks des ersten Fußes. Auch der abdrückende erste Fuß beschleunigt nach dem Abdruck, in vielen Fällen aber etwas später als der zweite Fuß (dies hängt aber von der Sportart und weiteren Faktoren ab). Wichtig ist aber, dass durch eine integrierte Analyse beider Beschleunigungswerte, die durch das beschriebene System ermöglicht wird, die Wirkung des Kraft-Einsatzes gemessen werden kann (vorausgesetzt der Läufer macht keine Fehler bei der Bewegung, was aber durch das System ermittelt und entsprechend korrigierend berücksichtigt werden kann). Durch diese Messung kann dann die Ermittlung des Krafteinsatzes durch das Gravitationsdruckpunktverfahren überprüft werden.

Die in der Beschreibung, den Ansprüchen und den Figuren offenbarten Merkmale der Erfindung können sowohl einzeln als auch in beliebiger Kombination für die Verwirklichung der Erfindung wesentlich sein.

## Patentansprüche

1. Tragbares Bewegungsanalysesystem zum Erfassen einer Bewegungsabweichung eines Nutzers von einem Bewegungsprofil für eine verbesserte Ausführung einer Sportart, mit folgenden Merkmalen:
ein erstes Sensormodul (100) mit zumindest einem ersten Beschleunigungssensor oder anderen Bewegungssensor (101), eine erste Datenübertragungseinheit (105) und eine erste Verarbeitungseinheit (102), wobei das erste Sensormodul (100) mit einer Extremität des Nutzers koppelbar ist und ausgebildet ist, um erste Beschleunigungswerte der Extremität als erste Sensordaten zu erfassen; und
ein zweites Sensormodul (200) mit zumindest einem zweiten Beschleunigungssensor oder anderen Bewegungssensor (201), eine zweite Datenübertragungseinheit (205) und eine zweite Verarbeitungseinheit (202), wobei das zweite Sensormodul (200) mit einer weiteren Extremität des Nutzers koppelbar ist und ausgebildet ist, um zweite Beschleunigungswerte der weiteren Extremität als zweite Sensordaten zu erfassen,
**dadurch gekennzeichnet, dass**
das erste Sensormodul (100) und das zweite Sensormodul (200) an oder in einer Sportausrüstung anbringbar sind und jeweils mit der Bewegung eines entsprechenden Beines des Nutzers koppelbar sind,
wobei die erste Verarbeitungseinheit (102) ausgebildet ist, um die erfassten Beschleunigungswerte von dem ersten Beschleunigungssensor (101) mit dem Bewegungsprofil zu vergleichen, und die erste Datenübertragungseinheit (105) ausgebildet ist, zumindest jene zu den ersten Beschleunigungswerten zugehörigen Daten an das zweite Sensormodul (200) zu übertragen, die von dem Bewegungsprofil abweichen,
und wobei die zweite Verarbeitungseinheit (202) ausgebildet ist, um die zweiten Beschleunigungswerte von dem zweiten Beschleunigungssensor (201) mit dem Bewegungsprofil zu vergleichen und mit den vom ersten Sensormodul (100) übertragenen Daten zu vergleichen, um dadurch die Sensordaten in einem Raum- und Zeitbezug zu setzen und eine Gesamtbeurteilung zu ermöglichen, und eine Bewegungsabweichung des Nutzers von dem Bewegungsprofil zu bestätigen und über die zweite Datenübertragungseinheit (205) auszugeben.

2. Bewegungsanalysesystem nach Anspruch 1, wobei die zweite Verarbeitungseinheit (202) ausgebildet ist, eine Bestätigung für die Bewegungsabweichung von dem Bewegungsprofil bezüglich des ersten Sensormoduls (100) und des zweiten Sensormoduls festzustellen und, in diesem Fall, eine Nachricht an das erste Sensormodul (100) zu senden, und wobei die erste Verarbeitungseinheit (102) ausgebildet ist, die Nachricht des zweiten Sensormoduls auszuwerten und das Bewegungsprofil anzupassen.

3. Bewegungsanalysesystem nach Anspruch 1 oder Anspruch 2, wobei die erste Verarbeitungseinheit (102) und die zweite Verarbeitungseinheit (202) ausgebildet sind, um eine zeitliche Abfolge von Beschleunigungsdaten mit deren Richtung und Neigungswinkel von dem ersten Beschleunigungssensor (101) und/oder von dem zweiten Beschleunigungssensor (201) zu erfassen und darauf basierend, eine Geschwindigkeit und/oder eine Richtungsänderung des Nutzers oder eines Sportgerätes oder einer Sportausrüstung festzustellen.

4. Bewegungsanalysesystem nach einem der vorhergehenden Ansprüchen, wobei das erste Sensormodul (100) und/oder das zweite Sensormodul (200) weiter zumindest eine der folgenden Komponenten aufweist: einen Datenspeicher (104, 204), einen Energiespeicher (103, 203), eine Nutzerschnittstelle (106, 206), einen Drucksensor (101d, 201d), einen Schwerkraftmesssensor, einen Temperatursensor, einen magnetischen Sensor (101b, 201b), ein Gyroskop, einen Windsensor, einen Vibrationssensor, eine 3D-Kamera (101c, 201c), eine Spektralanalyseeinheit, ein chemischer Analysesensor und eine Leitfähigkeitsmesseinrichtung, wobei der Drucksensor (101d, 201d) des ersten Sensormoduls (100) und/oder des zweiten Sensormoduls (200) ausgebildet ist, um einen Druck zwischen einem Sportausrüstung des Nutzers und einem Untergrund zu messen, und wobei die erste Verarbeitungseinheit (102) und/oder die zweite Verarbeitungseinheit (202) ausgebildet sind, um den gemessene Druck mit entsprechenden Werten in dem Bewegungsprofil zu vergleichen.

5. Bewegungsanalysesystem nach Anspruch 4, wobei die 3D-Kamera (101c) ausgebildet ist, um eine räumliche Abbildung einer Umgebung des ersten und/oder des zweiten Sensormoduls (100, 200) zu erzeugen, und die erste Verarbeitungseinheit (102) und/oder die zweite Verarbeitungseinheit (202) ausgebildet sind, um eine räumliche Orientierung einer Sportausrüstung im Vergleich zu einem Untergrund festzustellen und Abweichungen von einem Normwert auszugeben.

6. Bewegungsanalysesystem nach Anspruch 4 oder Anspruch 5, wobei die erste Verarbeitungseinheit (102) und/oder die zweite Verarbeitungseinheit (202) ausgebildet sind, um basierend auf Sensordaten von zumindest einen Beschleunigungssensor (101, 201), zumindest einem Gyroskop und/oder zumindest eine 3D-Kamara (101c, 201c) eine Traktion einer Sportausrüstung zu einem Untergrund festzustellen und Abweichungen von einem Normwert auszugeben.

7. Bewegungsanalysesystem nach einem der Ansprüche 4 bis 6, wobei die erste Verarbeitungseinheit (102) und/oder die zweite Verarbeitungseinheit (202) ausgebildet sind, um aus Messwerten in Bezug auf physikalischen und/oder chemischen Eigenschaften, insbesondere von der Leitfähigkeitsmessreinrichtung, eine Gleitfähigkeit von einem Sportgeräten auf gefrorenem Wasser, insbesondere Schnee, zu ermitteln, und wobei die erste Verarbeitungseinheit (102) und/oder die zweite Verarbeitungseinheit (202) ausgebildet sind, die Gleitfähigkeitsdaten zu erfassen und davon abhängig das Bewegungsprofil anzupassen.

8. Bewegungsanalysesystem nach einem der Ansprüche 4 bis 7, wobei die erste Verarbeitungseinheit (102) und/oder die zweite Verarbeitungseinheit (202) ausgebildet sind, aus Messwerten des Vibrationssensors Hinweise bezüglich eines Schnee- oder Rollwiderstandes zu einem Untergrund zu liefern.

9. Bewegungsanalysesystem nach einem der vorhergehenden Ansprüche, wobei die zweite Datenübertragungseinheit (205) ausgebildet ist, um ein Ergebnis der zweiten Verarbeitungseinheit (202) dem Nutzer zu visualisieren und/oder in einem Hauptspeicher (204) zu speichern und/oder zu einer Ausgabeeinheit zu senden.

10. Bewegungsanalysesystem nach einem der vorhergehenden Ansprüche, wobei die erste Verarbeitungseinheit (102) ausgebildet ist, um einen Bewegungsablauf eines Beines des Nutzers festzustellen, und die zweite Verarbeitungseinheit (202) ausgebildet ist, um einen Bewegungsablauf des anderen Beines des Nutzers festzustellen, und jeweils mit einer optimalen Form zu vergleichen und Abweichungen davon auszugeben,
und/oder wobei die zweite Verarbeitungseinheit (202) weiter ausgebildet ist, um Sensordaten, die von dem ersten und/oder dem zweiten Sensormodul (100, 200) erfasst wurden, zur direkten oder indirekten Messung einer Kraft und/oder eines Druckes zu nutzen, in einem zeitlichen Verlauf zu erfassen, mit einem Profil zu vergleichen und Abweichungen davon auszugeben,
wobei die indirekte Kraftmessung durch das Gravitationsdruckpunkt-Verfahren erfolgen kann.

11. Bewegungsanalysesystem nach einem der vorhergehenden Ansprüche, wobei die erste Verarbeitungseinheit (102) und/oder die zweite Verarbeitungseinheit (202) ausgebildet ist, um basierend auf den erfassten Daten ein für den Nutzer individuelles Bewegungsprofil zu erzeugen und für eine folgende Erfassung der Bewegungsabweichung des Nutzers zu verwenden, wobei die erfassten Daten Sensordaten von zumindest einem der vorhandenen Sensoren sind oder über die Nutzerschnittstelle (106, 206) von dem Nutzer eingegeben werden.

12. Bewegungsanalysesystem nach einem der vorhergehenden Ansprüche, wobei die erste Verarbeitungseinheit (102) und/oder die zweite Verarbeitungseinheit (202) ausgebildet ist, um basierend auf den erfassten Daten ein Belastungsprofil verschiedener Körperregionen und Muskelgruppen des Nutzers zu erstellen.

13. Sportausrüstung, insbesondere ein Skipaar oder ein Laufschuhpaar, mit einem Bewegungsanalysesystem nach einem der Ansprüche 1 bis 12.

14. Verfahren mit folgenden Schritten:
Erfassen von ersten Sensordaten, die von einer Bewegung eines Beines des Nutzers abhängen, durch ein erstes Sensormodul (100),
**gekennzeichnet durch**
Erfassen von zweiten Sensordaten, die von einer weiteren Bewegung eines weiteren Beines des Nutzers abhängen, durch ein zweites Sensormodul (200), wobei das erste Sensormodul (100) und das zweite Sensormodul (200) an oder in einer Sportausrüstung angeordnet sind;
Vergleichen der ersten Sensordaten mit einem Bewegungsprofil;
Übertragen der ersten Sensordaten von dem ersten Sensormodul (100) an das zweite Sensormodul (200), wenn die ersten Sensordaten von dem Bewegungsprofil abweichen;
Vergleichen der zweiten Sensordaten mit einem Bewegungsprofil;
Bestätigen einer Bewegungsabweichung des Nutzers von dem Bewegungsprofil; und
Ausgabe der Bestätigung.

15. Verfahren nach Anspruch 14, das weiter folgende Schritte umfasst:
Herstellen einer individualisierten Sportausrüstung basierend auf der erfassten Bewegungsabweichung, wobei das Herstellen eine individuelle Anpassung einer Dämpfung von Laufschuhen und/oder eine Anpassung einer Spannung von Skiern umfasst.

## Claims

1. A portable motion analysis system for detecting a deviation of a user movement from a motion profile for an improved exercising of a sport, the system comprising:
a first sensor module (100) with at least one first acceleration sensor or another motion sensor (101), a first data transmission unit (105) and a first processing unit (102), wherein the first sensor module (100) can couple to a body part of the user and is adapted to detect first acceleration values of the body part as first sensor data; and
a second sensor module (200) with at least one second acceleration sensor or another motion sensor (201), a second data transmission unit (205) and a second processing unit (202), wherein the second sensor module (200) can couple to a further part of the user and is configured to detect second acceleration values of the further body part as second sensor data,
**characterized in that**
the first sensor module (100) and the second sensor module (200) are installable or attachable to or in a sport equipment and each can couple to a motion of a corresponding leg of the user,
wherein the first processing unit (102) is adapted to compare the detected acceleration values from the first acceleration sensor (101) with the motion profile, and the first data transmission unit (105) is configured to transmit to the second sensor module (200) at least those data related to the first acceleration values that deviate from the motion profile,
and wherein the second processing unit (202) is configured to compare the second acceleration values of the second acceleration sensor (201) with the motion profile and with data transmitted from the first sensor module (100) to thereby put the sensor data in a spatial and temporal relation and to enable an overall assessment, and to confirm a movement deviation of the user from the motion profile and to output it over the second data transfer unit (205).

2. The portable motion analysis system according to claim 1, wherein the second processing unit (202) is configured to issue a confirmation for the movement deviation of the user from the motion profile in respect to the first sensor module (100) and the second sensor module and, in this case, to send a notice to the first sensor module (100), and wherein the first processing unit (102) is configured to evaluate the message of the second sensor module and to adapt the motion profile.

3. The motion analysis system according to claim 1 or claim 2, wherein the first processing unit (102) and the second processing unit (202) are configured to detect a time sequence of acceleration data together with a direction and inclination angle by the first acceleration sensor (101) and/or by the second acceleration sensor (201) and based thereon to determine a speed and/or a direction change of the user or a sporting equipment or a hardware.

4. The motion analysis system according to one of the preceding claims, wherein the first sensor module (100) and/or the second sensor module (200) comprise further at least one of the following components: a data storage (104, 204), an energy storage (103, 203), a user interface (106, 206), a pressure sensor (101d, 201d), a gravity measure sensor, a temperature sensor, a magnetic sensor (101b, 201b), a gyroscope, a wind sensor, a vibration sensor, a 3D camera (101c, 201c), a spectral analysis unit, a chemical analysis sensor and a conductivity measurement unit, wherein the pressure sensor (101d, 201d) of the first sensor module (100) and/or the second sensor module (200) are configured to measure a pressure between the sport equipment of the user and a ground, and wherein the first processing unit (102) and/or the second processing unit (202) are configured to compare the measured pressure with corresponding values of the motion profile.

5. The motion analysis system according to claim 4, wherein the 3D camera (101c) is configured to generate a spatial image of a surrounding of the first and/or the second sensor module (100, 200), and the first processing unit (102) and/or the second processing unit (202) are configured to detect a spatial orientation of the sport equipment in respect to a ground and to output deviations from a standard value.

6. The motion analysis system according to claim 4 or claim 5, wherein the first processing unit (102) and/or the second processing unit (202) are configured to detect based on sensor data of at least one acceleration sensor (101, 201), at least one gyroscope and/or at least one 3D camera (101c, 201c) a traction of the sport equipment in respect to the ground and to output deviations from a standard value.

7. The motion analysis system according to one of the claims 4 to 6, wherein the first processing unit (102) and/or the second processing unit (202) are configured to determine from measured values in respect to physical and/or chemical properties, in particular from the conductivity measurement unit, a sliding capacity of the sport equipment on frozen water, especially snow, and wherein the first processing unit (102) and/or the second processing unit (202) are configured to detect the sliding capacity and to adapt based thereon the motion profile.

8. The motion analysis system according to one of the claims 4 to 7, wherein the first processing unit (102) and/or the second processing unit (202) are configured to provide indications relating to the snow or rolling resistances on the ground from the measured values of the vibration sensors.

9. The motion analysis system according to one of the preceding claims, wherein the second data transmission unit (205) is configured to visualize a result of the second processing unit (202) to the user and/or to store the result in a main storage (204) and/or to send the result to an output unit.

10. Motion analysis system according to one of the preceding claims, wherein the first processing unit (102) is configured to detect a motion operation of a leg of the user and the second processing unit (202) is configured to detect a motion operation of another leg of the user, and to compare them with an optimal form and to output deviations therefrom,
and/or wherein the second processing unit (202) is further configured to use sensor data, which are detected by the first and/or the second sensor module (100, 200), for a direct or indirect measurement of a force and/or of a pressure, to put them in a time-like sequence, to compare them with a profile, and to output deviations therefrom, wherein the indirect force measurement is carried out by the gravitational pressure point method.

11. The motion analysis system according to one of the preceding claims, wherein the first processing unit (102) and/or the second processing unit (202) are configured to generate based on the detected data an individual motion profile for the user and to use it for a subsequent detection of the motion deviation of the user, wherein the detected data are sensor data of at least one of the available sensors or are input over the user interface (106, 206) by the user.

12. The motion analysis system according to one of the preceding claims, wherein the first processing unit (102) and/or the second processing unit (202) are configured to establish based on the detected data a stress profile of various body regions and muscle groups of the user.

13. Sport equipment, in particular a pair of skis or a pair of running shoes, with a motion analysis system according to one of the claims 1 to 12.

14. Method with the following steps:
detecting first sensor data by a first sensor module (100), that depends on a motion of a leg of the user,
**characterized by**
detecting of second sensor data which depends on a further movement of a further leg of the user by a second sensor module (200), wherein the first sensor module (100) and the second sensor module (200) are arranged at or in a sporting equipment;
comparing the first sensor data with a motion profile;
transmitting the first sensor data from the first sensor module (100) to the second sensor module (200), when the first sensor data deviates from the motion profile;
comparing the second sensor data with a motion profile;
confirming a motion deviation of the user from the motion profile; and
outputting the confirmation.

15. Method of claim 14, which further comprises the steps:
manufacturing an individual sporting equipment based on the detected motion deviation, wherein the manufacturing comprises individual adaptation of an attenuation of running shoes and/or an adaptation of a tension of skis.

## Revendications

1. Système portatif d'analyse de mouvement destiné à détecter un écart de mouvement d'un utilisateur par rapport à un profil de mouvement en vue d'améliorer l'exécution d'une discipline sportive, comprenant les caractéristiques suivantes :
un premier module de capteurs (100) comportant au moins un premier capteur d'accélération ou un autre capteur de mouvement (101), une première unité de transfert de données (105) et une première unité de traitement (102), le premier module de capteurs (100) pouvant être relié à un membre de l'utilisateur, étant conçu pour analyser des premières valeurs d'accélération du membre en tant que données du premier capteur ; et
un second module de capteurs (200) comportant au moins un deuxième capteur d'accélération ou un autre capteur de mouvement (201), une seconde unité de transfert de données (205) et une seconde unité de traitement (202), le second module de capteurs (200) pouvant être relié à un autre membre de l'utilisateur, et étant conçu pour analyser des valeurs d'accélération de l'autre membre en tant que données du deuxième capteur,
**caractérisé en ce que**
le premier module de capteurs (100) et le second module de capteurs (200) peuvent être fixés sur ou dans un équipement sportif et peuvent être respectivement couplés au mouvement de l'une et de l'autre jambe de l'utilisateur,
la première unité de traitement (102) étant conçue pour comparer les valeurs d'accélération recueillies par le premier capteur d'accélération (101) avec le profil de mouvement, et la première unité de transfert de données (105) étant conçue pour transmettre au second module de capteurs (200) au moins les données se rapportant aux premières valeurs d'accélération qui s'écartent du profil de mouvement,
et la seconde unité de traitement (202) étant conçue pour comparer les secondes valeurs d'accélération provenant du deuxième capteur d'accélération (201) avec le profil de mouvement et avec les données transmises par le premier module de capteurs (100) afin d'intégrer les données des capteurs dans un espace et un temps de référence, et permettre une appréciation globale et de confirmer un écart de mouvement de l'utilisateur par rapport au profil de mouvement et le délivrer à l'aide de la seconde unité de transfert de données (205).

2. Système d'analyse de mouvement selon la revendication 1, dans lequel la seconde unité de traitement (202) est conçue pour confirmer l'écart de mouvement par rapport au profil de mouvement en référence au premier module de capteurs (100) et au second module de capteurs et, dans ce cas, pour envoyer un message au premier module de capteurs (100), et dans lequel la première unité de traitement (102) est conçue pour évaluer le message du second module de capteurs et ajuster le profil de mouvement.

3. Système d'analyse de mouvement selon la revendication 1 ou la revendication 2, dans lequel la première unité de traitement (102) et la seconde unité de traitement (202) sont conçues pour détecter une séquence temporelle de données d'accélération, dont la direction et l'inclinaison, provenant du premier capteur d'accélération (101) et/ou du deuxième capteur d'accélération (201), et, à partir de ces données, pour déterminer une vitesse et/ou un changement de direction de l'utilisateur ou d'un matériel ou d'un équipement sportif.

4. Système d'analyse de mouvement selon l'une des revendications précédentes, dans lequel le premier module de capteurs (100) et/ou le second module de capteurs (200) comprennent en outre au moins l'un des composants suivants : une mémoire de données (104, 204), un accumulateur d'énergie (103, 203), une interface utilisateur (106, 206), un capteur de pression (101d, 201d), un capteur de mesure de la gravité, un capteur de température, un capteur magnétique (101b, 201b), un gyroscope, un capteur de vent, un capteur de vibrations, une caméra 3D (101c, 201c), une unité d'analyse spectrale, un capteur d'analyse chimique et un dispositif de mesure de la conductivité, le capteur de pression (101d, 201d) du premier module de capteurs (100) et/ou du second module de capteurs (200) étant conçu pour mesurer la pression entre un équipement sportif de l'utilisateur et un sol, et la première unité de traitement (102) et/ou la seconde unité de traitement (202) étant conçues pour comparer la pression mesurée avec les valeurs correspondantes dans le profil de mouvement.

5. Système d'analyse de mouvement selon la revendication 4, dans lequel la caméra 3D (101c) est conçue pour générer une représentation spatiale de l'environnement du premier et/ou du second module de capteurs (100, 200) et dans lequel la première unité de traitement (102) et/ou la seconde unité de traitement (202) sont conçues pour déterminer l'orientation spatiale d'un équipement sportif par rapport à un sol et pour délivrer en sortie des écarts par raport à une valeur de norme.

6. Système d'analyse de mouvement selon la revendication 4 ou la revendication 5, dans lequel la première unité de traitement (102) et/ou la seconde unité de traitement (202) sont conçues pour déterminer, sur la base des données d'au moins un capteur d'accélération (101, 201), d'au moins un gyroscope et/ou d'au moins une caméra 3D (101c, 201c), l'effort de traction d'un équipement de sport par rapport à un sol et pour délivrer en sortie les écarts par rapport à une valeur de norme.

7. Système d'analyse du mouvement selon l'une des revendications 4 à 6, dans lequel la première unité de traitement (102) et/ou la seconde unité de traitement (202) sont conçues pour déterminer, à partir de valeurs mesurées portant sur les propriétés physiques et/ou chimiques fournies notamment par le dispositif de mesure de la conductivité, l'aptitude au glissement d'un matériel de sport sur l'eau gelée, en particulier la neige, et dans lequel la première unité de traitement (102) et/ou la seconde unité de traitement (202) sont conçues pour recueillir les données d'aptitude au glissement et pour adapter le profil de mouvement en fonction de celles-ci.

8. Système d'analyse de mouvement selon l'une des revendications 4 à 7, dans lequel la première unité de traitement (102) et/ou la seconde unité de traitement (202) sont conçues pour fournir, à partir de valeurs de mesure du capteur de vibrations, des indications relatives à la résistance de la neige ou la résistance au roulement par rapport à un sol.

9. Système d'analyse de mouvement selon l'une des revendications précédentes, dans lequel la seconde unité de transfert de données (205) est conçue pour permettre à l'utilisateur de visualiser le résultat de la seconde unité de traitement (202) et/ou pour enregistrer ce résultat dans une mémoire centrale (204) et/ou pour l'envoyer vers une unité de sortie.

10. Système d'analyse de mouvement selon l'une des revendications précédentes, dans lequel la première unité de traitement (102) est conçue pour détecter le déroulement du mouvement type d'une jambes de l'utilisateur, et dans lequel la seconde unité de traitement (202) est conçue pour détecter le déroulement du mouvement de l'autre jambe de l'utilisateur, et afin de les comparer respectivement de manière optimale et de déduire les écarts par rapport à ceux-ci,
et/ou dans lequel la seconde unité de traitement (202) est en outre conçue pour utiliser les données recueillies par le premier et/ou le second module de capteurs (100, 200), en vue de mesurer directement ou indirectement un effort et/ou une pression, de les identifier dans un déroulement dans le temps, de les comparer à un profil et d'en déduire les écarts en résultant,
la mesure indirecte de l'effort pouvant être réalisée par la méthode du point d'application de la force de gravitation.

11. Système d'analyse de mouvement selon l'une des revendications précédentes, dans lequel la première unité de traitement (102) et/ou la seconde unité de traitement (202) sont conçues pour générer, sur la base des données recueillies, un profil de mouvement personnalisé de l'utilisateur et pour l'utiliser pour la détection ultérieure de l'écart de mouvement de l'utilisateur, les données recueillies étant des données de capteurs provenant d'au moins l'un des capteurs existants ou étant saisies par l'utilisateur via l'interface utilisateur (106, 206).

12. Système d'analyse de mouvement selon l'une des revendications précédentes, dans lequel la première unité de traitement (102) et/ou la seconde unité de traitement (202) sont conçues pour créer, sur la base des données recueillies, un profil de contrainte de différentes régions du corps et de groupes de muscles de l'utilisateur.

13. Matériel de sport, en particulier une paire de skis ou de chaussures de course, équipé d'un système d'analyse de mouvement selon l'une des revendications 1 à 12.

14. Procédé comprenant les étapes suivantes :
détection par un premier module de capteurs (100) de premières données qui dépendent du mouvement d'une jambe de l'utilisateur,
**caractérisé par**
la détection de secondes données de capteurs, qui dépendent du mouvement de l'autre jambe de l'utilisateur, par un second module de capteurs (200), le premier module capteur (100) et le second module capteur (200) étant disposés sur ou dans un équipement sportif ;
la comparaison des premières données avec un profil de mouvement ;
la transmission des première données de capteur provenant du premier module de capteurs (100) au second module de capteurs (200) lorsque les premières données de capteurs s'écartent du profil de mouvement ;
la comparaison des secondes données de capteurs avec un profil de mouvement ;
la confirmation d'un écart de mouvement de l'utilisateur par rapport au profil de mouvement ; et
la délivrance en sortie de la confirmation.

15. Procédé selon la revendication 14 comprenant en outre l'étape suivante :
la fabrication d'un matériel de sport personnalisé sur la base de l'écart de mouvement détectée, la fabrication comportant une adaptation personnalisée de l'amortissement des chaussures de course et/ou une adaptation à la tension des skis.
